# EUROPEAN PATENT SPECIFICATION

(11) **EP 3 065 753 B1**
(45) Date of publication and mention of the grant of the patent: **11.09.2019**
(21) Application number: 14833181.2
(22) Date of filing: 31.10.2014
(51) Int. Cl.: A61K 38/12, A61K 45/06, A61P 31/04

(54) **USE OF PEPTIDES IN ANTIBIOTIC RESISTANCE**
VERWENDUNG VON PEPTIDEN BEI ANTIBIOTIKARESISTENZ
UTILISATION DE PEPTIDES CONTRE LA RESISTANCE AUX ANTIBIOTIQUES

(30) Priority: 31.10.2013 US 201361898183 P; 18.11.2013 US 201361905440 P
(43) Date of publication of application: 14.09.2016
(60) Divisional application: 19189938.4
(73) Proprietor: Hutchison Biofilm Medical Solutions Limited, Nassau, New Providence (BS)
(72) Inventor: ZLOTKIN, Amir, 52621 Tel-Aviv (IL)
(74) Representative: Vossius & Partner Patentanwälte Rechtsanwälte mbB
(86) International application number: PCT/IB2014/002989
(87) International publication number: WO 2015/063608

(56) References cited:
- WO-A1-2010/076642
- WO-A2-02/061087
- WO-A2-2010/035107
- WO-A2-2012/164380

## Description

### FIELD OF THE INVENTION

The present invention relates to compositions and methods for increasing the effectiveness of antibiotics against bacteria, particularly antibiotic resistant bacteria.

### BACKGROUND OF THE INVENTION

Antibiotic use has become widespread and a cornerstone of medical treatment - being used to treat infections ranging from the seriously life-threatening to the more trivial and frequently non-bacterial illnesses. This constant antibiotic pressure, combined with the ability of bacteria to incorporate DNA from other strains and closely related species, has led to the evolution and acquisition of resistance traits. Multiple-antibiotic-resistant strains are now widespread and bacteria have developed at least one mechanism of resistance (and frequently many more) to every single antibiotic class. For example, Methicillin-resistant Staphylococcus aureus (MRSA) is one of the principal multi-drug resistant bacterial pathogens causing serious community and hospital-acquired infections, such as skin and soft tissue infections, bone, joint and implant infections, ventilator- associated pneumonia, and sepsis. It is estimated that multi-drug resistant *Staphylococcus aureus* infections leads to 19,000 deaths per year in the United States, with an associated 3-4 billion US dollars in additional annual health care costs. Despite this high mortality rate, there are relatively few new antibacterial agents in the pharmaceutical pipeline. Instead, the majority of antibiotics developed in the last decade are molecules re-engineered from existing antibiotic classes for which underlying resistance mechanisms are already present. Therefore effective new therapeutic options for treatment of infections caused, particularly those caused by multi-drug resistant bacteria are urgently needed.

Previously, it was shown that peptides found in a wide range of organisms across the animal kingdom can cause microorganisms to detach from surfaces and from each other, thus preventing the formation of biofilms. These proteins and peptides were not bactericidal and did not affect bacterial growth. WO 2012/164380, WO 2010/035107, WO 02/061087 or WO 2010/076642 do not disclose the use of an antibiotic and a cyclized peptide comprising the sequence SVHSFDYDWYNV for the treatment of a mammalian subject having a bacterial infection caused by an antibiotic resistant bacterial species.

The present inventors have found that the same peptide can increase the potency of an antibiotic against a bacterium, particularly an antibiotic resistant species, when used in combination with an antibiotic.

### SUMMARY OF THE INVENTION

The invention is as defined in the present claims. The present invention relates to an antibiotic and a cyclized peptide comprising a sequence SVHSFDYDWYNV for use in a method of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species. The present invention also relates to an antibiotic and a cyclized peptide comprising a sequence SVHSFDYDWYNV for use in a method of increasing the effectiveness of an antibiotic against an antibiotic resistant bacterium, comprising contacting the antibiotic resistant bacterium with the antibiotic and a cyclized peptide comprising the sequence SVHSFDYDWYNV , wherein the antibiotic and the peptide are administered to a subject in need thereof, wherein the subject is a mammal having a bacterial infection caused by the antibiotic resistant species.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of."

The phrase "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

The term "method" refers to manners, means, techniques and procedures for accomplishing a given task including, but not limited to, those manners, means, techniques and - procedures either known to, or readily developed from known manners, means, techniques and procedures by practitioners of the chemical, biological and biophysical arts.

As used herein the term "about" refers to ± 10 %.

As used herein, the term "bacteria killing concentration" of the antibiotic is the minimum concentration of the antibiotic at which a treated bacterial culture does not result in any bacterial colonies that can be cultured under appropriate conditions of culture medium and incubation. As used herein, the term "minimum bactericidal concentration" (MBC) of an antibiotic is the minimum or lowest concentration of the antibiotic required to kill a particular bacterium.

As used herein the term "contacting" refers to the positioning of the peptide to be used in accordance with the present invention and an antibiotic such that they are in direct or indirect contact with bacteria. Thus, the present invention contemplates both applying the compositions of the present invention to a desirable surface and/or directly to the bacterial cells. Contacting the compositions with a surface can be effected using any method known in the art including spraying, spreading, wetting, immersing, dipping, painting, ultrasonic welding, welding, bonding or adhering. Contacting the peptide and antibiotic with bacteria can also include administering the peptide and antibiotic to a subject in need thereof.

Other features and advantages of the invention will be apparent from the following detailed description, and from the claims.

### BRIEF DESCRIPTION OF THE DRAWINGS

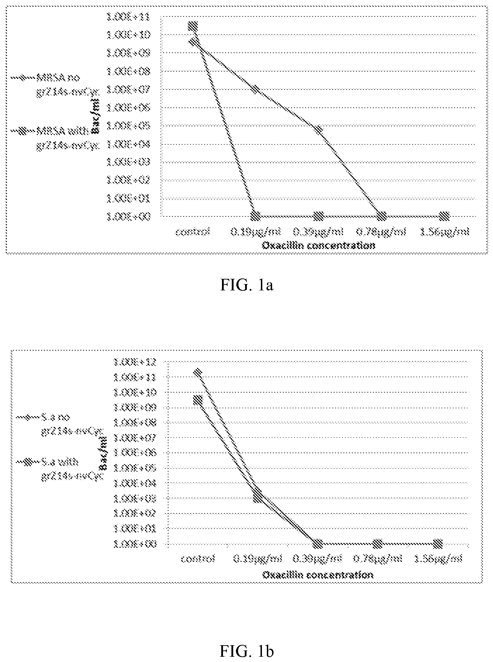

FIG. 1a demonstrates results from the testing of different concentrations of oxacillin, alone or with 10 nM grZ14s-nvCyc, against *S. aureus* ATCC 43300 (MRSA) (5×10⁵ CFU/mL). Figure 1b demonstrates results from the testing of different concentrations of oxacillin, alone or with 10 nM grZ14s-nvCyc, against *S. aureus* ATCC 25923 (MSSA) (5×10⁵ CFU/mL).

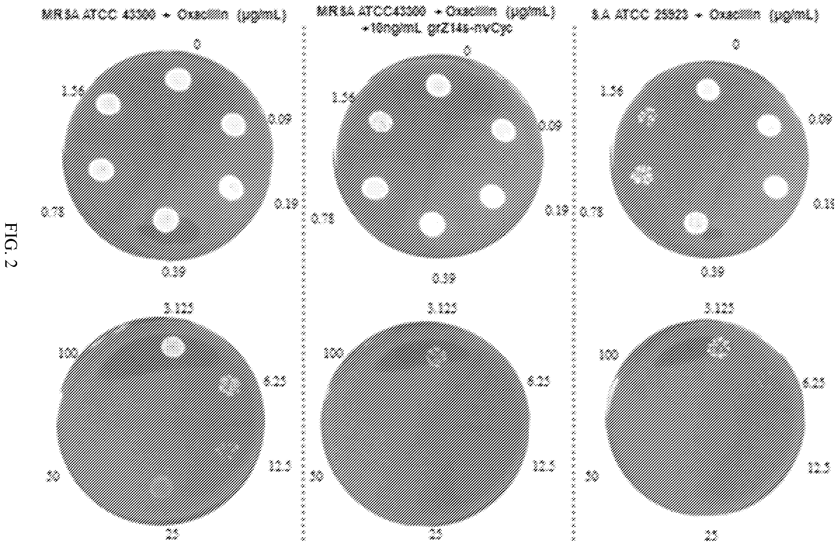

Figure 2 demonstrates results from the testing of different concentrations of oxacillin, alone or with 10 nM grZ14s-nvCyc, against *S. aureus* ATCC 43300 (MRSA) and *S. aureus* ATCC 25923 (MSSA), at bacterial concentrations of 7×10⁶ CFU/mL.

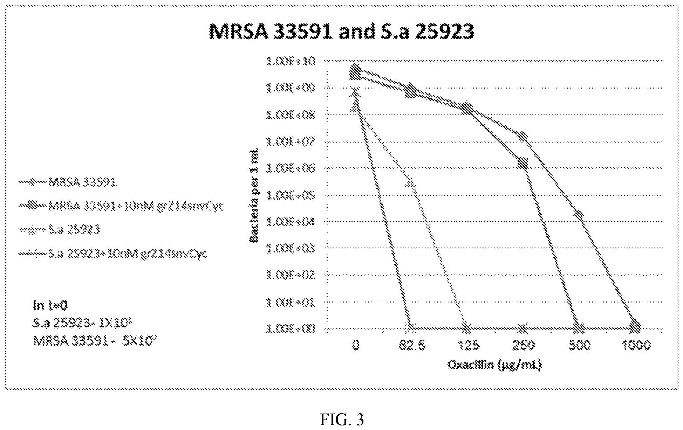

Figure 3 demonstrates results from the testing of different concentrations of oxacillin, alone or with 10 nM grZ14s-nvCyc, against *S. aureus* ATCC 33591 (MRSA strain) (5×10⁷ CFU/mL) and *S. aureus* ATCC 25923 (MSSA), at bacterial concentrations of 1×10⁸ CFU/mL.

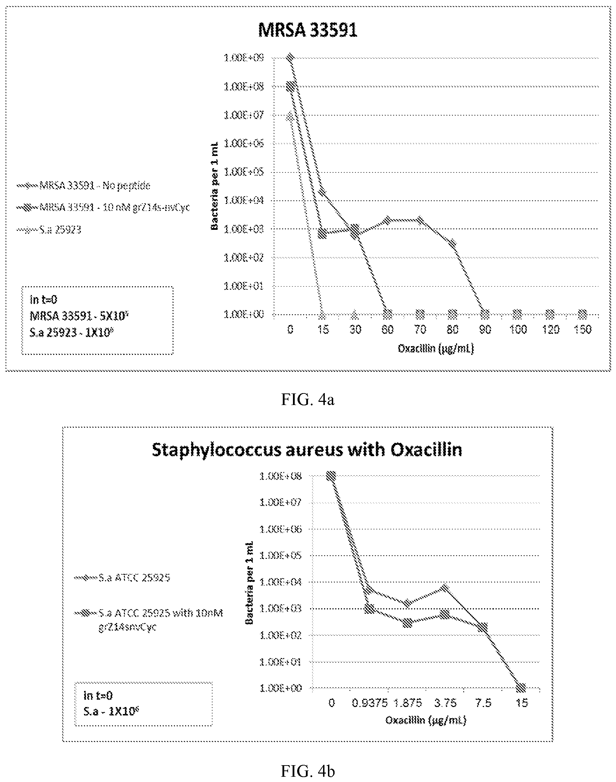

Figure 4a demonstrates results from the testing of different concentrations of oxacillin, alone or with 10 nM grZ14s-nvCyc, against *S. aureus* ATCC 33591 (MRSA strain) (5×10⁵ CFU/mL) and activity of oxacillin alone on *S. aureus* ATCC 25923 (MSSA), at bacterial concentrations of 1×10⁶ CFU/mL. Figure 4b demonstrates results from the testing of different concentrations of oxacillin, alone or with 10 nM grZ14s-nvCyc, against *S. aureus* ATCC 25923 (1×10⁶ CFU/mL).

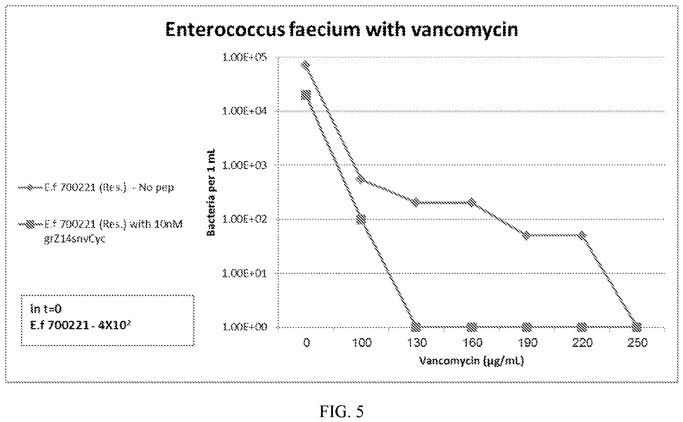

Figure 5 demonstrates results from the testing of different concentrations of vancomycin, alone or with 10 nM grZ14s-nvCyc, against *Enterococcus faecium* ATCC 700221 (vancomycin, teicoplanin and tetracyclin resistant strain) (4×10² CFU/mL).

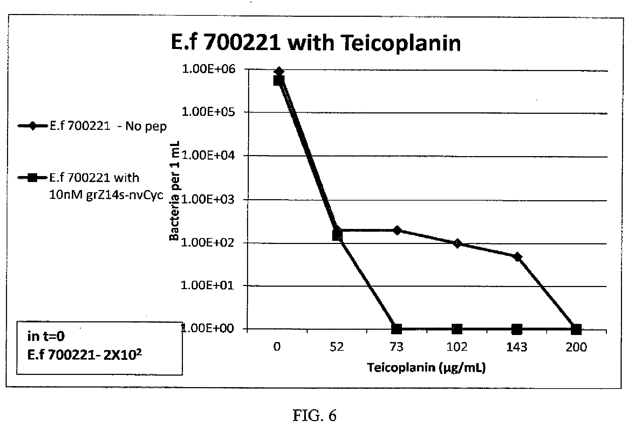

Figure 6 demonstrates results from the testing of different concentrations of teicoplanin, alone or with 10 nM grZ14s-nvCyc, against *Enterococcus faecium* ATCC 700221 (vancomycin, teicoplanin and tetracyclin resistant strain) (2×10² CFU/mL).

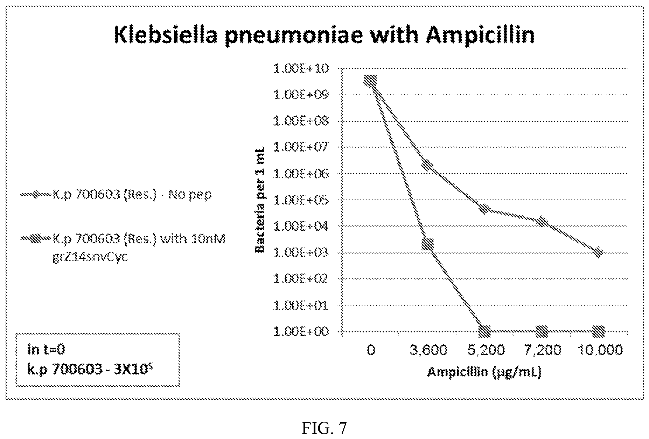

Figure 7 demonstrates results from the testing of different concentrations of ampicillin, alone or with 10 nM grZ14s-nvCyc, against *Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (3×10⁵ CFU/mL).

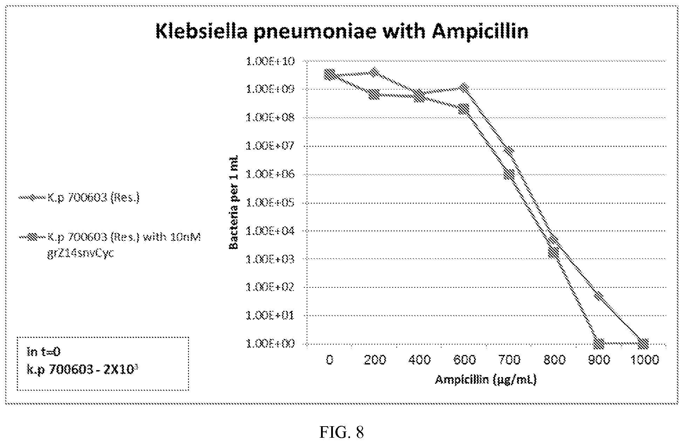

Figure 8 demonstrates results from the testing of different concentrations of ampicillin, alone or with 10 nM grZ14s-nvCyc, against *Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (2×10³ CFU/mL).

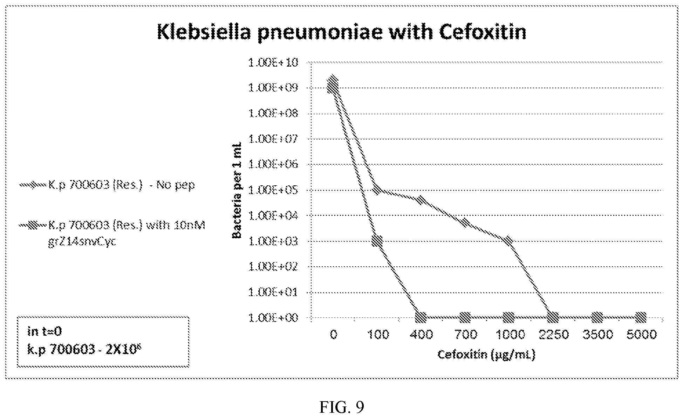

Figure 9 demonstrates results from the testing of different concentrations of cefoxitin, alone or with 10 nM grZ 14s-nvCyc, against *Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (2×10⁶ CFU/mL).

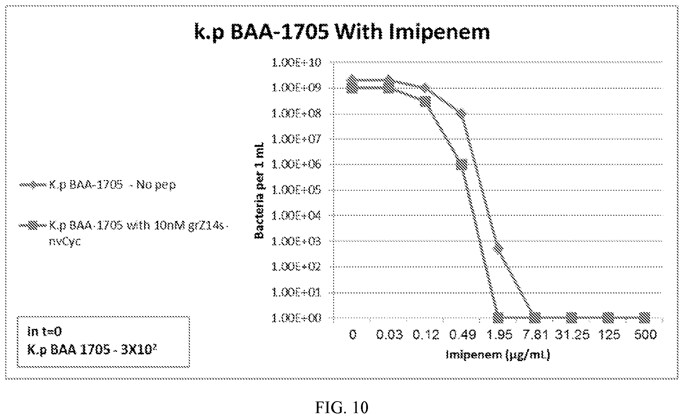

Figure 10 demonstrates results from the testing of different concentrations of imipenem, alone or with 10 nM grZ 14s-nvCyc, against *Klebsiella pneumoniae* ATCC BAA® 1705 (carbapenem - imipenem and ertapenem, amikacin, aztreonam, levofloxacin and ciprofloxacin - resistant strain) (3×10² CFU/mL).

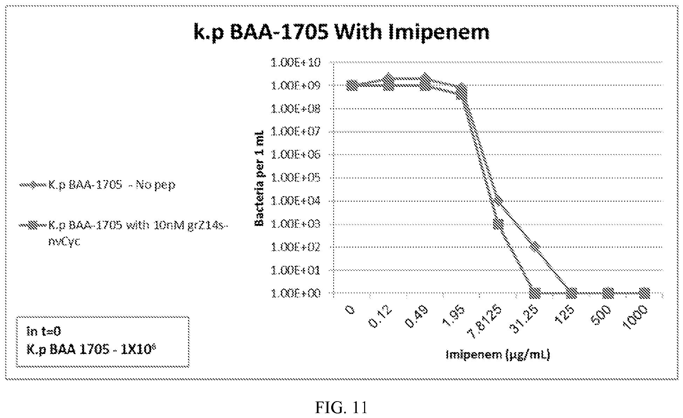

Figure 11 demonstrates results from the testing of different concentrations of imipenem, alone or with 10 nM grZ14s-nvCyc, against *Klebsiella pneumoniae* ATCC BAA® 1705 (carbapenem - imipenem and ertapenem, amikacin, aztreonam, levofloxacin and ciprofloxacin - resistant strain) (1×10⁶ CFU/mL).

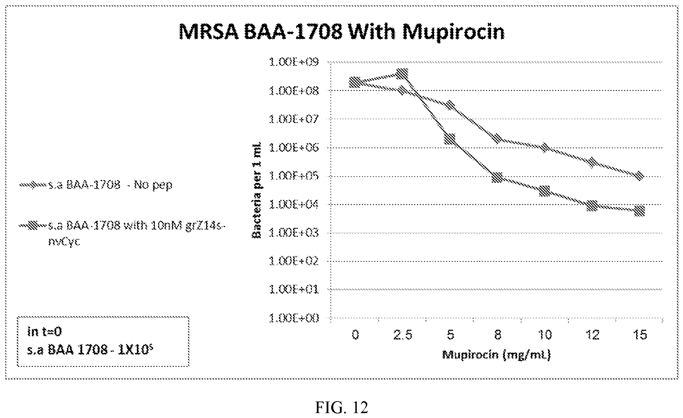

Figure 12 demonstrates results from the testing of different concentrations of mupirocin, alone or with 10 nM grZ14s-nvCyc, against *S. aureus* ATCC BAA® 1708 (MRSA-mupA positive strain) (1×10⁵ CFU/mL).

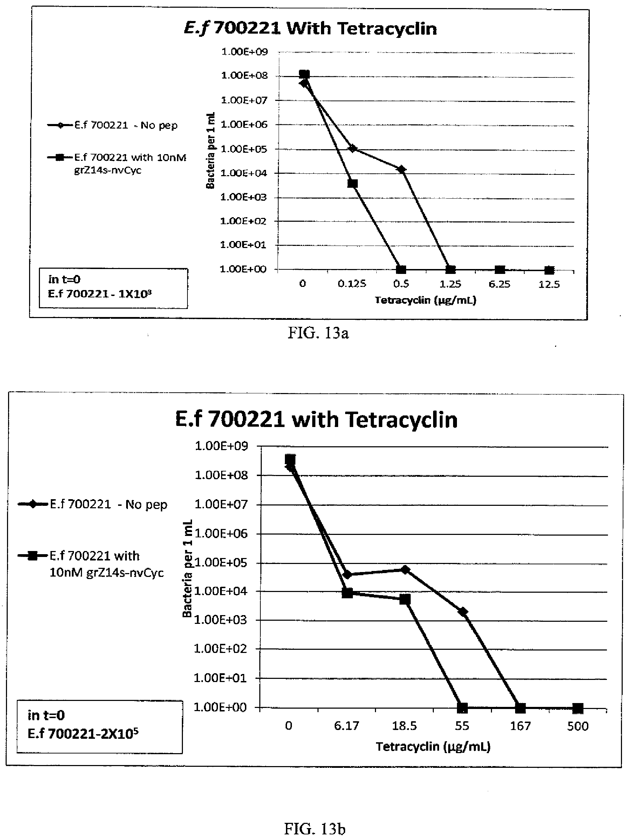

Figure 13a demonstrates results from the testing of different concentrations of tetracyclin, alone or with 10 nM grZ14s-nvCyc, against *Enterococcus faecium* ATCC 700221 (vancomycin, teicoplanin and tetracyclin resistant strain) (1×10³ CFU/mL). Figure 13b demonstrates results from the testing of different concentrations of tetracyclin, alone or with 10 nM grZ14s-nvCyc, against *Enterococcus faecium* ATCC 700221 (vancomycin, teicoplanin and tetracyclin resistant strain) (2×10⁵ CFU/mL).

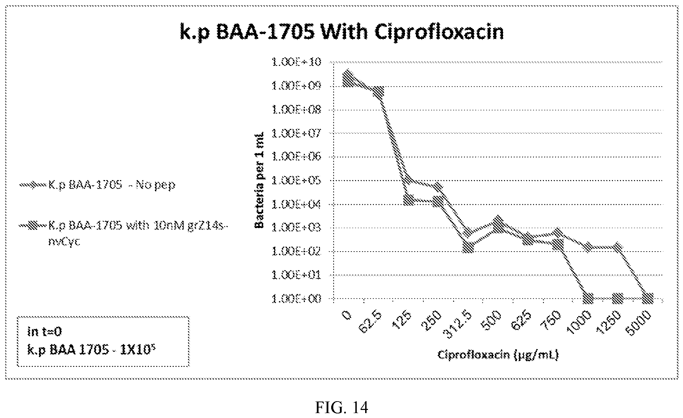

Figure 14 demonstrates results from the testing of different concentrations of ciprofloxacin, alone or with 10 nM grZ14s-nvCyc, against *Klebsiella pneumoniae* ATCC BAA® 1705 (carbapenem - imipenem and ertapenem, amikacin, aztreonam, levofloxacin and ciprofloxacin - resistant strain) (1×10⁵ CFU/mL).

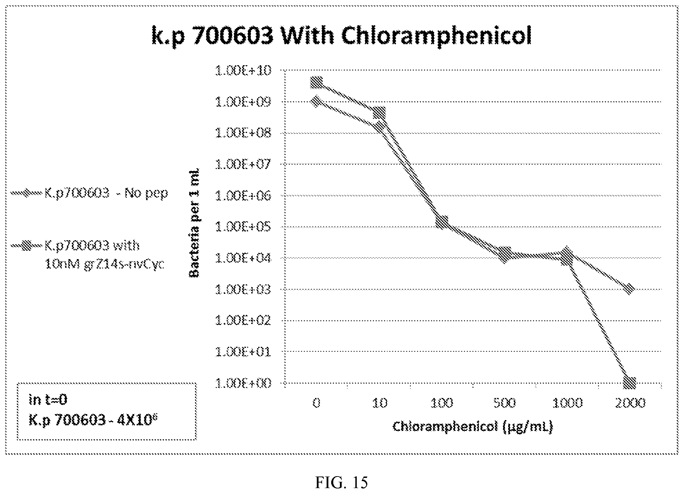

Figure 15 demonstrates results from the testing of different concentrations of chloramphenicol, alone or with 10 nM grZ14s-nvCyc, against *Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (4×10⁶ CFU/mL)

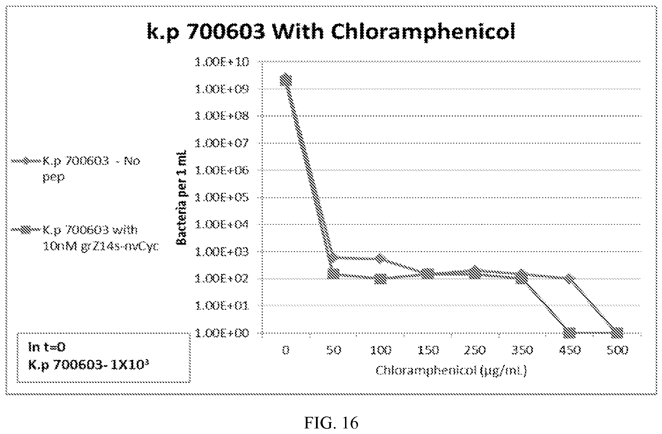

Figure 16 demonstrates results from the testing of different concentrations of chloramphenicol, alone or with 10 nM grZ 14s-nvCyc, against *Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (1×10³ CFU/mL).

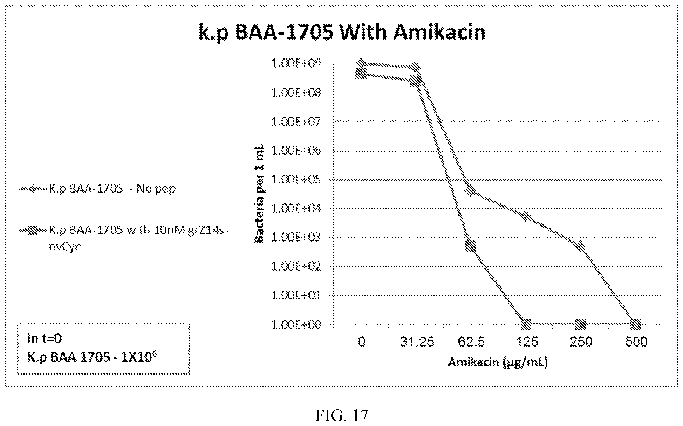

Figure 17 demonstrates results from the testing of different concentrations of amikacin, alone or with 10 nM grZ14s-nvCyc, against *Klebsiella pneumoniae* ATCC BAA® 1705 (carbapenem - imipenem and ertapenem, amikacin, aztreonam, levofloxacin and ciprofloxacin - resistant strain) (1×10⁶ CFU/mL).

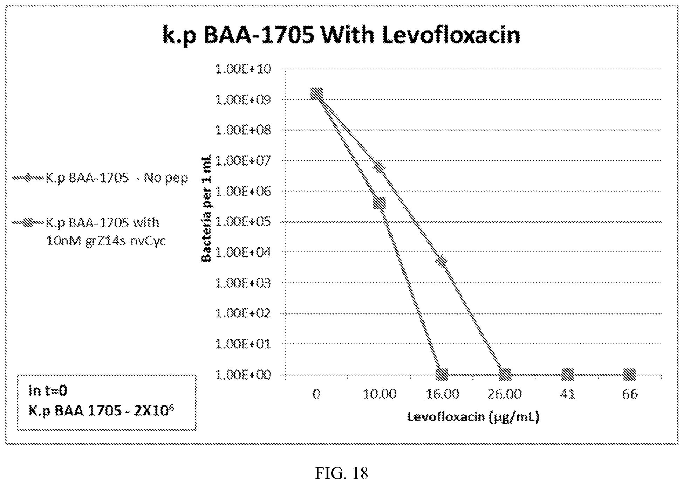

Figure 18 demonstrates results from the testing of different concentrations of levofloxacin, alone or with 10 nM grZ 14s-nvCyc, against *Klebsiella pneumoniae* ATCC BAA® 1705 (carbapenem - imipenem and ertapenem, amikacin, aztreonam, levofloxacin and ciprofloxacin - resistant strain) (2×10⁶ CFU/mL).

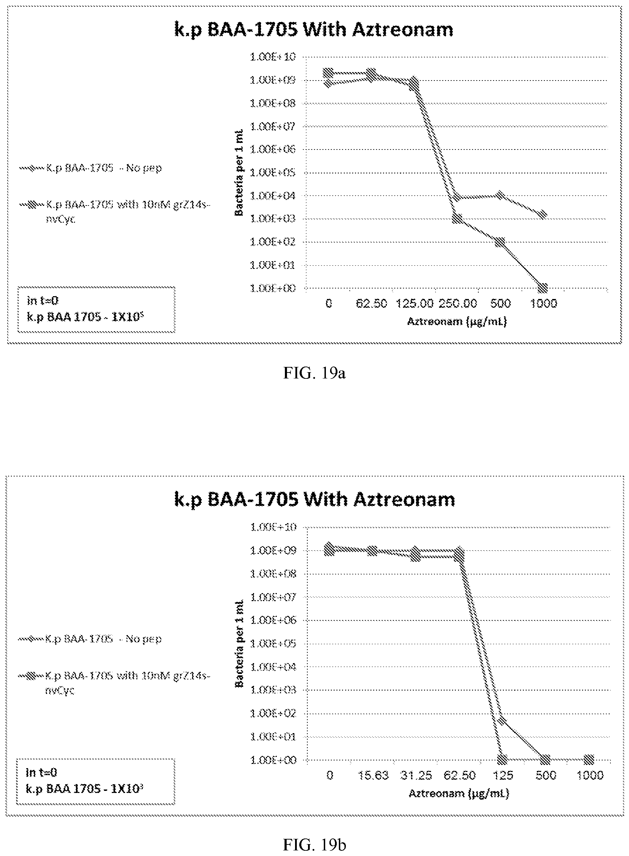

Figure 19a demonstrates results from the testing of different concentrations of aztreonam, alone or with 10 nM grZ 14s-nvCyc, against *Klebsiella pneumoniae* ATCC BAA® 1705 (carbapenem - imipenem and ertapenem, amikacin, aztreonam, levofloxacin and ciprofloxacin - resistant strain) (1×10⁵ CFU/mL). Figure 19b demonstrates results from the testing of different concentrations of aztreonam, alone or with 10 nM grZ14s-nvCyc, against *Klebsiella pneumoniae* ATCC BAA® 1705 (1×10³ CFU/mL).

### DETAILED DESCRIPTION

The invention relates to an antibiotic and a cyclized peptide comprising a sequence SVHSFDYDWYNV for use in a method described herein of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

In some embodiments, the invention is an antibiotic and a cyclized peptide comprising a sequence SVHSFDYDWYNV for use in a method of increasing the potency of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

In some embodiments, the increase in potency or increase in effectiveness of the antibiotic is measured by analyzing the minimum bactericidal concentration of the antibiotic against the bacterium. Thus, in some embodiments, the invention is an antibiotic and a cyclized peptide comprising a sequence SVHSFDYDWYNV for use in a method of increasing the effectiveness of an antibiotic against a bacterium, wherein the minimum bactericidal concentration of the antibiotic against the bacterium is decreased by at least about 5%, about 10%, about 15%, about 20%, about 25%, about 30%, about 35%, about 40%, about 45%, about 50%, about 55%, about 60%, about 65%, about 70%, about 75%, about 80%, about 85% or about 90%. The percent decrease in the minimum bactericidal concentration (MBC) is measured by dividing the difference between the MBC with and without a peptide of the invention by the MBC without the peptide of the invention and multiplying the result by 100.

In some embodiments, the minimum bactericidal concentration of the antibiotic against the bacterium is decreased from about 5-25%, from about 10-30%, from about 20-60%, from about 20-40%, from about 20-50%, from about 40-90%, from about 5-90%, from about 10-80%, from 20-70%, from about 30-60%, or from about 30-50%.

In some embodiments, the increase in potency or increase in the effectiveness of the antibiotic is measured by analyzing the bacteria killing concentration of the antibiotic against the bacterium.

In some embodiments, the increase in potency or increase in effectiveness of the antibiotic is measured by measuring the bacteria killing concentration of the antibiotic. Thus, in some embodiments, the invention is an antibiotic and a cyclized peptide comprising a sequence SVHSFDYDWYNV for use in a method of increasing the effectiveness of an antibiotic against a bacterium, wherein the bacteria killing concentration of the antibiotic against the bacterium is decreased by at least about 5%, about 10%, about 15%, about 20%, about 25%, about 30%, about 35%, about 40%, about 45%, about 50%, about 55%, about 60%, about 65%, about 70%, about 75%, about 80%, about 85% or about 90%. The percent decrease in the bacteria killing concentration is measured by dividing the difference between the bacteria killing concentration with and without a peptide of the invention by the bacteria killing concentration without the peptide of the invention and multiplying the result by 100.

In some embodiments, the bacteria killing concentration of the antibiotic against the bacterium is decreased from about 5-25%, from about 10-30%, from about 20-60%, from about 20-40%, from about 20-50%, from about 40-90%, from about 5-90%, from about 10-80%, from 20-70%, from about 30-60%, or from about 30-50%.

### Peptides

The methods described herein comprise contacting a bacterium with an antibiotic and a cyclized peptide comprising the sequence SVHSFDYDWYNV. For example, the peptide may comprise at least one of SVHSFDYDWYNV, SVHSFDYDWYNVS, KSVHSFDYDWYNVS, KSVHSFDYDWYNVSD, NKSVHSFDYDWYNVSD, NKSVHSFDYDWYNVSDQ, QNKSVHSFDYDWYNVSDQ, QNKSVHSFDYDWYNVSDQA, SQNKSVHSFDYDWYNVSDQ, SQNKSVHSFDYDWYNVSDQA, FSQNKSVHSFDYDWYNVSDQA, FSQNKSVHSFDYDWYNVSDQAD, SFSQNKSVHSFDYDWYNVSDQAD, SFSQNKSVHSFDYDWYNVSDQADL, SFSQNKSVHSFDYDWYNVSDQADLK, SFSQNKSVHSFDYDWYNVSDQADLKN, or CSFSQNKSVHSFDYDWYNVSDQADLKNC or combinations thereof.

According to some embodiments, the peptide comprises part of a sequence comprising up to about 20, up to about 25, up to about 30, up to about 40, or up to about 50 amino acids.

In some embodiments, the peptides for use in accordance with the present invention have a two cysteines in the sequence. In some embodiments, the cysteines are at the C-terminal and the N-terminal.

The peptides for use in accordance with the present invention are cyclized (i.e., in cyclic form) and are indicated in this application as such with the term "cyc." In one embodiment, each peptide is modified with a cysteine at the C-terminal and a cysteine at the N-terminal, wherein the C- and N-terminals are S-S bridged. In specific embodiments, one or more of the peptides are modified with a cysteine at the C-terminal and a cysteine at the N-terminal, wherein the C- and N-terminals are S-S bridged. In other embodiments, the peptides are cyclized internally, for example, when one or more amino acids in the peptide that are not at the N-terminal or the C-terminal are linked to each other. Such a linkage may be via a S-S bridge between two cysteines, or may be a chemically synthesized covalent linkage, for example, an ester or an amide group.

In some embodiments, the peptides for use in accordance with the present invention may optionally comprise at least two sequences, connected by a linker of some type, such that the N-terminal of a first peptide sequence is connected to the C-terminal of the linker, and the C-terminal of a second peptide sequence is connected to the N-terminal of the linker.

In some embodiments, the peptide has the sequence CSVHSFDYDWYNVC. In some embodiments, the peptide CSVHSFDYDWYNVC is cyclized via the two cysteines at the C-terminal and the N-terminal.

In some embodiments, the peptide is soluble. In other embodiments, the peptide is attached to a linker. In some embodiments, the linker is polyethylene glycol or palmitic acid. In some embodiments, the peptide is synthetic.

### Bacterial species

In some embodiments of the antibiotic and the cyclized peptide comprising a sequence SVHSFDYDWYNV for use in a method in accordance with the invention, the bacterium is a gram positive species. In some embodiments, the bacterium is a gram negative species.

The term "Gram-positive bacteria" as used herein refers to bacteria characterized by having as part of their cell wall structure peptidoglycan as well as polysaccharides and/or teichoic acids and are characterized by their blue-violet color reaction in the Gram-staining procedure. Representative Gram-positive bacteria include: *Actinomyces* spp., *Bacillus anthracis, Bifidobacterium* spp., *Clostridium botulinum, Clostridium perfringens,*
*Clostridium* spp., *Clostridium tetani*, *Corynebacterium diphtheriae*, *Corynebacterium jeikeium*, *Enterococcus faecalis, Enterococcus faecium, Erysipelothrix rhusiopathiae, Eubacterium* spp., *Gardnerella vaginalis, Gemella morbillorum, Leuconostoc* spp., *Mycobacterium abscessus*, *Mycobacterium avium complex, Mycobacterium chelonae, Mycobacterium fortuitum*, *Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia* spp., *Peptococcus niger, Peptostreptococcus* spp., *Proprionibacterium* spp., *Sarcina lutea, Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (group B *streptococcus*), *Streptococcus anginosus, Streptococcus bovis, Streptococcus canis, Streptococcus equi, Streptococcus milleri, Streptococcus mitior, Streptococcus mutans, Streptococcus pneumoniae*, *Streptococcus pyogenes* (group A *streptococcus*), *Streptococcus salivarius, Streptococcus sanguis.*

The term "Gram-negative bacteria" as used herein refers to bacteria characterized by the presence of a double membrane surrounding each bacterial cell. Representative Gram-negative bacteria include *Acinetobacter calcoaceticus, Acinetobacter baumannii, Actinobacillus actinomycetemcomitans, Aeromonas hydrophila, Alcaligenes xylosoxidans, Bacteroides, Bacteroides fragilis, Bartonella bacilliformis, Bordetella* spp., *Borrelia burgdorferi, Branhamella catarrhalis, Brucella* spp., *Campylobacter* spp., *Chalmydia pneumoniae, Chlamydia psittaci, Chlamydia trachomatis, Chromobacterium violaceum,*
*Citrobacter* spp., *Eikenella corrodens, Enterobacter aerogenes, Escherichia coli, Flavobacterium meningosepticum, Fusobacterium* spp., *Haemophilus influenzae, Haemophilus* spp., *Helicobacter pylori, Klebsiella pneumoniae,*
*Klebsiella* spp., *Legionella* spp., *Leptospira* spp., *Moraxella catarrhalis, Morganella morganii, Mycoplasma pneumoniae, Neisseria gonorrhoeae, Neisseria meningitidis, Pasteurella multocida, Plesiomonas shigelloides, Prevotella*spp*., Proteus* spp., *Providencia rettgeri, Pseudomonas aeruginosa, Pseudomonas* spp., *Rickettsia prowazekii, Rickettsia rickettsii, Rochalimaea* spp., *Salmonella* spp., *Salmonella typhi, Serratia marcescens,*
*Shigella* spp., *Shigella sonnei, Treponema carateum, Treponema pallidum, Treponema pallidum endemicum, Treponema pertenue, Veillonella* spp.,*Vibrio cholerae, Vibrio vulnifìcus, Yersinia enterocolitica, Yersinia pestis.*

In some embodiments, the bacterium exhibits non-specific resistance to antibiotics, for example, by the formation of biofilms. In some embodiments, the bacterium exhibits specific resistance to particular antibiotics. The specific resistance in the bacteria may be either innate or acquired. In some embodiments, the bacterium exhibits a both specific and non-specific resistance to antibiotics. In some embodiments, the bacteria are present in very low concentrations, and therefore do not form biofilms. Without limiting the invention, it is hypothesized that bacteria that are not in biofilms exhibit specific resistance mechanisms to antibiotics. Without limiting the invention, it is also hypothesized that the peptides for use in accordance with the present invention act in countering these specific non-biofilm related resistance mechanisms.

In some embodiments, the bacterium is *Staphylococcus aureus.* In particular embodiments, the *S. aureus* is a methicillin resistant species (MRSA).

In some embodiments, the bacterium is *Enterococcus faecium.* In some embodiments, the *E. faecium* is a vancomycin-resistant species. In other embodiments, the *E. faecium* is a teicoplanin-resistant species. In some embodiments, the *E. faecium* is a vancomycin- and teicoplanin-resistant species. In some embodiments, the *E. faecium* is resistant to the tetracyclin group of antibiotics.

In some embodiments, the bacterium is *Klebsiella pneumoniae.* In some embodiments, the *K. pneumonia* is an antibiotic resistant species. In particular embodiments, the *K. pneumoniae* is resistant to the penicillin group of antibiotics. In particular embodiments, the *K. pneumoniae* is resistant to the cephalosporin group of antibiotics. In particular embodiments, the *K. pneumoniae* is resistant to the carbapenem group of antibiotics. In particular embodiments, the *K. pneumoniae* is resistant to the fluoroquinolone group of antibiotics. In particular embodiments, the *K. pneumoniae* is resistant to protein synthesis inhibitor antibiotics. In particular embodiments, the *K. pneumoniae* is resistant to ampicillin, aztreonam, cefoxitin, cefpodoxime, ceftazidime, chloramphenicol, piperacillin, and tetracycline. In particular embodiments, the *K. pneumoniae* is resistant to carbapenem - imipenem and ertapenem, amikacin, aztreonam, levofloxacin, and ciprofloxacin.

### Antibiotics

The invention relates to an antibiotic and a cyclized peptide comprising a sequence SVHSFDYDWYNV for use in a method of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species. In some embodiments, the invention is an antibiotic and a cyclized peptide comprising a sequence SVHSFDYDWYNV for use in a method of increasing the potency of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

In some embodiments, the antibiotic is a cell envelope antibiotic, nucleic acid inhibitor, or protein synthesis inhibitor.

In some embodiments, the antibiotic is a cell envelope antibiotic selected from the group consisting of penicillins, cephalosporins, glycopeptides, and carbapenems. In some embodiments, the antibiotic is a penicillin selected from the group consisting of oxacillin, methicillin, amoxicillin, ampicillin, cloxacillin, dicloxacillin, carbenicillin, ticarcillin, and piperacillin. In some embodiments, the antibiotic is a cephalosporin selected from the group consisting of cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, loracarbef, cefbuperazone, cefmetazole, cefminox, cefotetan, cefoxitin, cefotiam, cefclidine, cefepime, cefluprenam, cefoselis, cefozopran, cefpirome, cefquinome, flomoxef, ceftobiprole, and ceftaroline. In some embodiments, the antibiotic is a glycopeptide selected from the group consisting of vancomycin and teicoplanin. In some embodiments, the antibiotic is a carbapenem selected from the group consisting of imipenem, morepenem, ertapenem, doripenem, panipenem, and biapenem. In particular embodiments, the antibiotic is aztreonam.

In some embodiments, the antibiotic is a nucleic acid inhibitor selected from the group consisting of fluoroquinolones. In some embodiments, the fluoroquinolone is ciprofloxacin, enoxacin, fleroxacin, lomefloxacin, nadifloxacin, norfloxacin, ofloxacin, pefloxacin, rufloxacin, balofloxacin, grepafloxacin, levofloxacin, temafloxacin, tosufloxacin, clinafloxacin, gatifloxacin, gemifloxacin, moxifloxacin, sitafloxacin, trovafloxacin, prolufloxacin, delafloxacin, danofloxacin, difloxacin, enrofloxacin, ibafloxacin, marbofloxacin, orbifloxacin, or sarafloxacin. In particular embodiments, the fluroquinolone is ciprofloxacin or levofloxacin.

In some embodiments, the antibiotic is a protein synthesis inhibitor selected from the group consisting of chloramphenicol and amikacin. In other embodiments, the antibiotic is a protein synthesis inhibitor selected from the macrolide class of antibiotics. Macrolide antibiotics include azithromycin, clarithromycin, erythromycin, telithromycin, carbomycin A, josamycin, kitasamycin, midecamycin, oleandomycin, solithromycin, spiramycin, troleandomycin, roxithromycin, telithromycin, cethromycin, solithromycin, spiramycin, or ansamycin. In some embodiments, the protein synthesis inhibitor antibiotic is mupirocin.

In some embodiments, the antibiotic is one having an unknown mechanism of action.

In particular embodiments, the antibiotic exhibits reduced activity against particular species of bacteria due to specific or nonspecific resistance by the bacteria against the antibiotic.

### Administration

The invention relates to an antibiotic and a cyclized peptide comprising a sequence SVHSFDYDWYNV for use in a method of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species. In some embodiments, the invention is an antibiotic and a cyclized peptide comprising a sequence SVHSFDYDWYNV for use in a method of increasing the potency of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

In some embodiments, the bacterium is contacted with the antibiotic and the peptide simultaneously, i.e., at the same time. Such simultaneous contact of the antibiotic and the peptide can be accomplished, for example, by using a single composition containing both the antibiotic and the peptide. In other embodiments, the simultaneous contact of the antibiotic and the peptide can be accomplished by contacting the bacterium with the antibiotic and the peptide in two separate compositions.

In some embodiments, the bacterium is contacted with the antibiotic and the peptide at different times. In particular embodiments, the antibiotic and the peptide are contacted with the bacterium about 15 minutes apart, about 30 minutes apart, about 45 minutes apart, about 1 hour apart, about 1.5 hours apart, or about 2 hours apart. In some embodiments where the bacterium is contacted with the antibiotic and the peptide at different times, the bacterium may be contacted with the peptide before or after contacting the bacterium with the antibiotic.

In some embodiments, the invention is an antibiotic and a cyclized peptide comprising a sequence SVHSFDYDWYNV for use in a method of increasing the potency of an antibiotic against a bacterium, wherein the bacterium is an antibiotic-resistant species, comprising administering the antibiotic and a cyclized peptide comprising the sequence SVHSFDYDWYNV to a subject in need thereof, wherein the subject is a mammal having a bacterial infection caused by the antibiotic resistant species. In some embodiments, the subject is a non-human mammal, for example, dog, cat, horses, pigs, cows. In other embodiments, the mammal is a human.

In some embodiments the cyclized peptide administered to the subject may comprise at least one of SVHSFDYDWYNV, SVHSFDYDWYNVS, KSVHSFDYDWYNVS, KSVHSFDYDWYNVSD, NKSVHSFDYDWYNVSD, NKSVHSFDYDWYNVSDQ, QNKSVHSFDYDWYNVSDQ, QNKSVHSFDYDWYNVSDQA, SQNKSVHSFDYDWYNVSDQ, SQNKSVHSFDYDWYNVSDQA, FSQNKSVHSFDYDWYNVSDQA, FSQNKSVHSFDYDWYNVSDQAD, SFSQNKSVHSFDYDWYNVSDQAD, SFSQNKSVHSFDYDWYNVSDQADL, SFSQNKSVHSFDYDWYNVSDQADLK, SFSQNKSVHSFDYDWYNVSDQADLKN, or CSFSQNKSVHSFDYDWYNVSDQADLKNC, or combinations thereof. In particular embodiments, the peptide has the sequence CSVHSFDYDWYNVC.

According to some embodiments, the peptide comprises part of a sequence comprising up to about 20, up to about 25, up to about 30, up to about 40, or up to about 50 amino acids.

In some embodiments, the peptides for use in accordance with the present invention have a two cysteines in the sequence. In some embodiments, the cysteines are at the C-terminal and the N-terminal.

The peptides for use in accordance with the present invention are cyclized (i.e., in cyclic form) and are indicated in this application as such with the term "cyc." In one embodiment, each peptide is modified with a cysteine at the C-terminal and a cysteine at the N-terminal, wherein the C- and N-terminals are S-S bridged. In specific embodiments, one or more of the peptides are modified with a cysteine at the C-terminal and a cysteine at the N-terminal, wherein the C- and N-terminals are S-S bridged. In other embodiments, the peptides are cyclized internally, for example, when one or more amino acids in the peptide that are not at the N-terminal or the C-terminal are linked to each other. Such a linkage may be via a S-S bridge between two cysteines, or may be a chemically synthesized covalent linkage, for example, an ester or an amide group.

In some embodiments, the peptides for use in accordance with the present invention may optionally comprise at least two sequences, connected by a linker of some type, such that the N-terminal of a first peptide sequence is connected to the C-terminal of the linker, and the C-terminal of a second peptide sequence is connected to the N-terminal of the linker.

In some embodiments, the peptide has the sequence CSVHSFDYDWYNVC. In some embodiments, the peptide CSVHSFDYDWYNVC is cyclized via the two cysteines at the C-terminal and the N-terminal.

In some embodiments, the peptide is soluble. In other embodiments, the peptide is attached to a linker. In some embodiments, the linker is polyethylene glycol or palmitic acid. In some embodiments, the peptide is synthetic.

In some embodiments, the antibiotic is a cell envelope antibiotic, nucleic acid inhibitor, or protein synthesis inhibitor. In some embodiments, the antibiotic is one of the antibiotics as described above. In particular embodiments, the antibiotic is ampicillin, oxacillin, cefoxitin, vancomycin, teicoplanin, imipenem, ertrapenem, mupirocin, tetracyclin, ciprofloxacin, levofloxacin, chloramphenicol, amikacin, or aztreonam.

In particular embodiments, the antibiotic exhibits reduced activity against particular species of bacteria due to specific or nonspecific resistance by the bacteria against the antibiotic.

The mammal has a bacterial infection in need of treatment, wherein the bacterial infection is caused by an antibiotic resistant bacterium. In some embodiments, the bacterium exhibits non-specific resistance to antibiotics, for example, by the formation of biofilms. In some embodiments, the bacterium exhibits specific resistance to particular antibiotics. The specific resistance in the bacteria may be either innate or acquired. In some embodiments, the bacterium exhibits a both specific and non-specific resistance to antibiotics.

In some embodiments, the bacterium is a gram positive species. In some embodiments, the bacterium is a gram negative species. Examples of gram positive and gram negative species are as defined above.

In some embodiments, the bacterium is *Staphylococcus aureus.* In particular embodiments, the *S. aureus* is a methicillin resistant species (MRSA).

In some embodiments, the bacterium is *Enterococcus faecium.* In some embodiments, the *E. faecium* is a vancomycin-resistant species. In other embodiments, the *E. faecium* is a teicoplanin-resistant species. In some embodiments, the *E. faecium* is a vancomycin- and teicoplanin-resistant species. In some embodiments, the *E. faecium* is resistant to the tetracyclin group of antibiotics.

In some embodiments, the bacterium is *Klebsiella pneumoniae.* In some embodiments, the *K. pneumoniae* is an antibiotic resistant species. In particular embodiments, the *K. pneumoniae* is resistant to the penicillin group of antibiotics. In particular embodiments, the *K. pneumoniae* is resistant to the cephalosporin group of antibiotics. In particular embodiments, the *K. pneumoniae* is resistant to the carbapenem group of antibiotics. In particular embodiments, the *K. pneumoniae* is resistant to the fluoroquinolone group of antibiotics. In particular embodiments, the *K. pneumoniae* is resistant to protein synthesis inhibitor antibiotics. In particular embodiments, the *K. pneumoniae* is resistant to ampicillin, aztreonam, cefoxitin, cefpodoxime, ceftazidime, chloramphenicol, piperacillin, and tetracycline. In particular embodiments, the *K. pneumoniae* is resistant to carbapenem - imipenem and ertapenem, amikacin, aztreonam, levofloxacin, and ciprofloxacin.

In some embodiments, the antibiotic and the peptide are administered to the subject simultaneously, *i.e.,* at the same time. Such simultaneous administration of the antibiotic and the peptide can be accomplished, for example, by using a single composition containing both the antibiotic and the peptide. In other embodiments, the simultaneous administration of the antibiotic and the peptide can be accomplished by administering the antibiotic and the peptide in two separate compositions.

In some embodiments, the antibiotic and the peptide are administered to the subject at different times. In particular embodiments, the antibiotic and the peptide are administered about 15 minutes apart, about 30 minutes apart, about 45 minutes apart, about 1 hour apart, about 1.5 hours apart, or about 2 hours apart. In some embodiments where the antibiotic and the peptide are administered at different times, the peptide may be administered before or after the antibiotic.

### Compositions

In some embodiments, the invention is a composition, comprising an antibiotic and a cyclized peptide comprising a sequence SVHSFDYDWYNV for use in a method of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

In some embodiments, the cyclized peptide comprising a sequence SVHSFDYDWYNV may have any of the sequences as described above. In particular embodiments, the peptide has the sequence CSVHSFDYDWYNVC. In some embodiments, the peptide CSVHSFDYDWYNVC is cyclized via the two cysteines at the C-terminal and the N-terminal.

In some embodiments, the antibiotic is a cell envelope antibiotic, nucleic acid inhibitor, or protein synthesis inhibitor. In some embodiments, the antibiotic is one of the antibiotics as described above. In particular embodiments, the antibiotic is ampicillin, oxacillin, cefoxitin, vancomycin, teicoplanin, imipenem, ertrapenem, mupirocin, tetracyclin, ciprofloxacin, levofloxacin, chloramphenicol, amikacin, or aztreonam.

In some embodiments, the peptide and antibiotic to be contacted with the bacterium are in a single composition. In some embodiments, the peptide and antibiotic to be contacted with the bacterium are in two separate compositions.

Compositions of the disclosure include devices coated with the peptide and an antibiotic as described above. Exemplary devices which are contemplated by the present invention include, but are not limited to, vessel hulls, automobile surfaces, air plane surfaces, membranes, filters, and industrial equipment. The surface may also be comprised in medical devices, instruments, and implants.

Examples of such medical devices, instruments, and implants include any object that is capable of being implanted temporarily or permanently into a mammalian organism, such as a human. Representative medical devices, instruments, and implants that may be used according to the present invention include, for example, central venous catheters, urinary catheters, endotracheal tubes, mechanical heart valves, pacemakers, vascular grafts, stents and prosthetic joints.

Medical devices that may be coated according to the teachings of the present disclosure include, but are not limited to, artificial blood vessels, catheters and other devices for the removal or delivery of fluids to patients, artificial hearts, artificial kidneys, orthopedic pins, prosthetic joints, plates and implants; catheters and other tubes (including urological and biliary tubes, endotracheal tubes, peripherally insertable central venous catheters, dialysis catheters, long term tunneled central venous catheters, peripheral venous catheters, short term central venous catheters, arterial catheters, pulmonary catheters, Swan-Ganz catheters, urinary catheters, peritoneal catheters), urinary devices (including long term urinary devices, tissue bonding urinary devices, artificial urinary sphincters, urinary dilators), shunts (including ventricular or arterio-venous shunts); prostheses (including breast implants, penile prostheses, vascular grafting prostheses, aneurysm repair devices, mechanical heart valves, artificial joints, artificial larynxes, otological implants), anastomotic devices, vascular catheter ports, vascular stents, clamps, embolic devices, wound drain tubes, ocular lenses, dental implants, hydrocephalus shunts, pacemakers and implantable defibrillators, needleless connectors, voice prostheses and the like. Another possible application of the present disclosure is the coating of surfaces found in the medical and dental environment. Such surfaces include the inner and outer aspects of various instruments and devices, whether disposable or intended for repeated uses. Such surfaces include the entire spectrum of articles adapted for medical use, including without limitation, scalpels, needles, scissors and other devices used in invasive surgical, therapeutic or diagnostic procedures; blood filters. Other examples will be readily apparent to practitioners in these arts.

Surfaces found in the medical environment also include the inner and outer aspects of pieces of medical equipment, medical gear worn or carried by personnel in the health care setting. Such surfaces can include surfaces intended as biological barriers to infectious organisms in medical settings, such as gloves, aprons and face shields. Commonly used materials for biological barriers are thermoplastic or polymeric materials such as polyethylene, dacron, nylon, polyesters, polytetrafluoroethylene, polyurethane, latex, silicone and vinyl. Other surfaces can include counter tops and fixtures in areas used for medical procedures or for preparing medical apparatus, tubes and canisters used in respiratory treatments, including the administration of oxygen, of solubilized drugs in nebulizers and of anesthetic agents. Other such surfaces can include handles and cables for medical or dental equipment not intended to be sterile. Additionally, such surfaces can include those non-sterile external surfaces of tubes and other apparatus found in areas where blood or body fluids or other hazardous biomaterials are commonly encountered. The compositions of the present disclosure can be used on the surface of or within these medical devices to provide long term protection against colonization by single cell organisms and reduce the incidence of device-related infections.

The compositions of the present disclosure can be directly incorporated into the polymeric matrix of the medical device at the polymer synthesis stage or at the device manufacture stage. The compositions can also be covalently attached to the medical device polymer. These and many other methods of coating medical devices are evident to one of ordinary skill in the art.

Additional surfaces that can be treated according to the teachings of the present disclosure include the inner and outer aspects of those articles involved in water purification, water storage and water delivery, and those articles involved in food processing. Thus the present invention envisions coating a solid surface of a food or beverage container to extend the shelf life of its contents.

Surfaces related to health can also include the inner and outer aspects of those household articles involved in providing for nutrition, sanitation or disease prevention. Thus, the compositions of the present disclosure can be used for removal of disease-causing microorganisms from external surfaces. These can include, for example food processing equipment for home use, materials for infant care, tampons, soap, detergents, health and skincare products, household cleaners and toilet bowls.

The surface may be also be laboratory articles including, but not limited to, microscopic slide, a culturing hood, a Petri dish or any other suitable type of tissue culture vessel or container known in the art.

Underwater surfaces include any water immersed surface, including ships'/boats's hulls (i.e., the body or frame of a ship or boat), submergence vehicles, navigational aids, screens, nets, constructions, floating or emplaced offshore platforms (e.g., docks), buoys, signaling equipment and articles which come into contact with sea water or salty water. Other underwater surfaces include structures exposed to sea water including pilings, marine markers, undersea conveyances like cabling and pipes, fishing nets, bulkheads, cooling towers, and any device or structure that operates submerged.

The compositions of the present disclosure can be incorporated into marine coatings to limit undesirable marine biofouling. Thus, the anti-biofouling agents of the present disclosure can be formulated so as not to contain toxic materials (such as heavy metals), and still retain their efficacy. The anti-biofouling paint of the present disclosure may further contain binders(s), pigment(s), solvent(s) and additive(s).

Examples of solvents that may be used include aromatic hydrocarbons such as xylene and toluene; aliphatic hydrocarbons such as hexane and heptane, esters such as ethyl acetate and butyl acetate; amides such as N-methylpyrrolidone and N,N-dimethylformamide; alcohols such as isopropyl alcohol and butyl alcohol; ethers such as dioxane, THF and diethyl ether; and ketones such as methyl ethyl ketone, methyl isobutyl ketone and methyl isoamyl ketone. The solvents may be used alone or in combination thereof.

Examples of binders that may be used include alkyd resin, acrylic or vinyl emulsions, polyurethane resins, epoxy resins, silicone based resins, acrylic resins, inorganic silicate based resins, vinyl resins, particularly a vinyl chloride/vinyl acetate copolymer, and rosin. Examples of pigments that may be used include titanium dioxide, cuprous oxide, iron oxide, talc, aluminum flakes, mica flakes, ferric oxide, cuprous thiocyanate, zinc oxide, cupric acetate meta-arsenate, zinc chromate, zinc dimethyl dithiocarbamate, zinc ethylene bis(dithiocarbamate) and zinc diethyl dithiocarbamate.

Examples of additives that may be incorporated into the coating composition include dehumidifiers, wetting/dispersing agents, anti-settling agents, anti-skinning agents, drying/curing agents, anti-marring agents and additives ordinarily employed in coating compositions as stabilizers and anti-foaming agents.

Methods of preparing marine anti-biofouling paints are explained in detail in U.S. Pat. No. 4,678,512; U.S. Pat. No. 4,286,988; U.S. Pat. No. 4,675,051 ; U.S. Pat. No. 4,865,909; and U.S. Pat. No. 5,143,545.

The compositions of the present disclosure may also be used for providing antibacterial properties in cosmetics, to prevent spoiling of the product.

The compositions may further be used to provide an antibacterial effect to the mouth, teeth and gums, such as by incorporation in a toothpaste, mouthwash, or chewing gum.

Pharmaceutical compositions for use in accordance with the present invention may be administered by any suitable route, for example, include oral, rectal, transmucosal, especially transnasal, intestinal, or parenteral delivery, including intramuscular, subcutaneous, and intramedullary injections, as well as intrathecal, direct intraventricular, intravenous, intraperitoneal, intranasal, or intraocular injections.

Alternately, one may administer the composition for use in accordance with the present invention in a local rather than systemic manner, for example, via injection of the composition directly into a tissue region of a patient.

Pharmaceutical compositions for use in accordance with the present invention may be manufactured by processes well known in the art, e.g., by means of conventional mixing, dissolving, granulating, dragee-making, levigating, emulsifying, encapsulating, entrapping, or lyophilizing processes. Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For topical administration, the compositions for use in accordance with the present invention may be formulated as a gel, a cream, a wash, a rinse or a spray.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, and sodium carbomethylcellulose; and/or physiologically acceptable polymers such as polyvinylpyrrolidone (PVP). If desired, disintegrating agents, such as cross- linked polyvinyl pyrrolidone, agar, or alginic acid or a salt thereof, such as sodium alginate, may be added.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions, and suitable organic solvents or solvent mixtures. Dyestuffs or pigments may be added to the tablets or dragee coatings for identification or to characterize different combinations of active compound doses. Pharmaceutical compositions that can be used orally include push-fit capsules made of gelatin, as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate, and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofiuoromethane, dichloro-tetrafluoroethane, or carbon dioxide. In the case of a pressurized aerosol, the dosage may be determined by providing a valve to deliver a metered amount. Capsules and cartridges of, for example, gelatin for use in a dispenser may be formulated containing a powder mix of the compound and a suitable powder base, such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with, optionally, an added preservative. The compositions may be suspensions, solutions, or emulsions in oily or aqueous vehicles, and may contain formulatory agents such as suspending, stabilizing, and/or dispersing agents.

Pharmaceutical compositions for parenteral administration include aqueous solutions of the active preparation in water-soluble form. Additionally, suspensions of the active ingredients may be prepared as appropriate oily or water-based injection suspensions.

Suitable lipophilic solvents or vehicles include fatty oils such as sesame oil, or synthetic fatty acid esters such as ethyl oleate, triglycerides, or liposomes. Aqueous injection suspensions may contain substances that increase the viscosity of the suspension, such as sodium carboxymethyl cellulose, sorbitol, or dextran.

Optionally, the suspension may also contain suitable stabilizers or agents that increase the solubility of the active ingredients, to allow for the preparation of highly concentrated solutions. Alternatively, the active ingredient may be in powder form for constitution with a suitable vehicle, e.g., a sterile, pyrogen-free, water-based solution, before use.

In some embodiments, a composition for use in accordance with the invention can be delivered in an immediate release form. In other embodiments, a composition for use in accordance with the invention can be delivered in a controlled-release system or sustained-release system. Controlled- or sustained-release pharmaceutical compositions can have a common goal of improving drug therapy over the results achieved by their non-controlled or non-sustained-release counterparts. Advantages of controlled- or sustained-release compositions include extended activity of the antibiotic and the peptide, reduced dosage frequency, and increased compliance. In addition, controlled- or sustained-release compositions can favorably affect the time of onset of action or other characteristics, such as blood levels of the antibiotic and the peptide, and can thus reduce the occurrence of adverse side effects.

Controlled- or sustained-release compositions can comprise an immediate release portion and an extended release portion. The immediate release portion initially immediately releases an amount of the peptide and/or the antibiotic that promptly produces the desired therapeutic or prophylactic effect, while the extended release portion gradually and continually releases other amounts of the peptide and/or the antibiotic to maintain a level of therapeutic or prophylactic effect over an extended period of time. Controlled- or sustained-release of an active ingredient can be stimulated by various conditions, including but not limited to, changes in pH, changes in temperature, concentration or availability of enzymes, concentration or availability of water, or other physiological conditions or compounds.

Controlled-release and sustained-release dosage forms can be used to provide controlled- or sustained-release of one or more active ingredients using, for example, hydroxypropylmethyl cellulose, other polymer matrices, gels, permeable membranes, osmotic systems, multilayer coatings, microparticles, multiparticulates, liposomes, microspheres, or a combination thereof to provide the desired release profile in varying proportions. Suitable controlled- or sustained-release formulations known in the art, including those described herein, can be readily selected for use with the active ingredients of the invention in view of this disclosure. See also Goodson, "Dental Applications" (pp. 115-138) in Medical Applications of Controlled Release, Vol. 2, Applications and Evaluation, R. S. Langer and D. L. Wise eds., CRC Press (1984). Other controlled- or sustained-release systems that are discussed in the review by Langer, Science 249:1527-1533 (1990) can be selected for use according to the present invention. In one embodiment, a pump can be used (Langer, Science 249:1527-1533 (1990); Sefton, CRC Crit. Ref Biomed. Eng. 14:201 (1987); Buchwald et al., Surgery 88:507 (1980); and Saudek et al., N. Engl. J. Med 321:574 (1989)). In another embodiment, polymeric materials can be used (see Medical Applications of Controlled Release (Langer and Wise eds., 1974); Controlled Drug Bioavailability, Drug Product Design and Performance (Smolen and Ball eds., 1984); Ranger and Peppas, J. Macromol. Sci. Rev. Macromol. Chem. 23:61 (1983); Levy et al., Science 228:190 (1985); During et al., Ann. Neurol. 25:351 (1989); and Howard et al., J. Neurosurg. 71:105 (1989)). In yet another embodiment, a controlled- or sustained-release system can be placed in proximity of a target of infection, e.g., skin, lungs, spinal column, brain, or gastrointestinal tract, thus requiring only a fraction of the systemic dose.

When in tablet or pill form, a pharmaceutical composition for use in accordance with the invention can be coated to delay disintegration and absorption in the gastrointestinal tract, thereby providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions can include standard excipients such as mannitol, lactose, starch, magnesium stearate, sodium saccharin, cellulose, and magnesium carbonate. In one embodiment, the excipients are of pharmaceutical grade.

The pharmaceutical composition for use in accordance with the present invention may also be formulated in rectal compositions such as suppositories or retention enemas, using, for example, conventional suppository bases such as cocoa butter or other glycerides.

Pharmaceutical compositions suitable for use in the context of the present invention include compositions wherein the active ingredients are contained in an amount effective to achieve the intended purpose. More specifically, a "therapeutically effective amount" means an amount of active ingredients (e.g., peptide and antibiotic) effective to prevent, alleviate, or ameliorate symptoms of a pathogenic infection (e.g., fever) or prolong the survival of the subject being treated. Determination of a therapeutically effective amount is well within the capability of those skilled in the art, especially in light of the detailed disclosure provided herein.

For any preparation for use in the methods in accordance with the invention, the dosage or the therapeutically effective amount can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition. (See, e.g., Fingl, E. et al. (1975), "The Pharmacological Basis of Therapeutics," Ch. 1, p.l.) Dosage amount and administration intervals may be adjusted individually to provide sufficient plasma or brain levels of the active ingredient to induce or suppress the biological effect (i.e., minimally effective concentration, MEC or minimum bactericidal concentration, MBC). The MEC will vary for each preparation, but can be estimated from in vitro data. Dosages necessary to achieve the MEC will depend on individual characteristics and route of administration. Detection assays can be used to determine plasma concentrations.

Suitable effective dosage amounts can range from about 0.01 mg/kg of body weight to about 3000 mg/kg of body weight of the patient per day, although they are typically from about 0.01 mg/kg of body weight to about 2500 mg/kg of body weight of the patient per day or from about 0.01 mg/kg of body weight to about 1000 mg/kg of body weight of the patient per day. In one embodiment, the effective dosage amount is about 100 mg/kg of body weight of the patient per day or less. In another embodiment, the effective dosage amount ranges from about 0.01 mg/kg of body weight to about 100 mg/kg of body weight of the patient per day of the antibiotic or peptide, in another embodiment, about 0.02 mg/kg of body weight to about 50 mg/kg of body weight of the patient per day, and in another embodiment, about 0.025 mg/kg of body weight to about 20 mg/kg of body weight of the patient per day.

Administration can be as a single dose or as a divided dose. In one embodiment, an effective dosage amount is administered or contacted with the bacterium about every 24 hours until the condition is abated. In another embodiment, an effective dosage amount is administered or contacted with the bacterium about every 12 hours until the condition is abated. In another embodiment, an effective dosage amount is administered or contacted with the bacterium about every 8 hours until the condition is abated. In another embodiment, an effective dosage amount is administered or contacted with the bacterium about every 6 hours until the condition is abated. In another embodiment, an effective dosage amount is administered or contacted with the bacterium about every 4 hours until the condition is abated. The effective dosage amounts described herein refer to amounts of each of the antibiotic or peptide administered.

Depending on the severity and responsiveness of the condition to be treated, dosing can be of a single or a plurality of administrations, with course of treatment lasting from several days to several weeks, or until cure is effected or diminution of the disease state is achieved.

The amount of a composition to be administered will, of course, be dependent on the subject being treated, the severity of the affliction, the manner of administration, the judgment of the prescribing physician, etc.

Compositions for use in accordance with the present invention may, if desired, be presented in a pack or dispenser device, such as an FDA-approved kit, which may contain one or more unit dosage forms containing the active ingredient. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation for use in accordance with the invention formulated in a pharmaceutically acceptable carrier may also be prepared, placed in an appropriate container, and labeled for treatment of an indicated condition, as further detailed above.

Pharmaceutical compositions for use in accordance with the invention include single unit dosage forms suitable for oral administration such as, but not limited to, tablets, capsules, gelcaps, and caplets that are adapted for controlled- or sustained-release.

The following examples are illustrative, but not limiting, of the an antibiotic and a cyclized peptide comprising a sequence SVHSFDYDWYNV for use in methods in accordance with the present invention. Suitable modifications and adaptations of the variety of conditions and parameters normally encountered in the treatment of bacterial infections and which are obvious to those skilled in the art in view of this disclosure are within the invention.

### EXAMPLES

### Example 1 - Activity of oxacillin and a peptide comprising FDYDWY Against methicillin resistant and methicillin sensitive S. aureus

*Staphylococcus aureus* ATCC 43300 (5×10⁵ CFU/mL) (MRSA strain) was incubated in 1.5 mL Eppendorf tubes with either oxacillin alone or oxacillin with the grZ14s-nvCyc peptide (10nM) (cyclized CSVHSFDYDWYNVC) for 24 hours at 37°C with shaking (250 rpm). The solutions in the tubes were diluted ten-fold with phosphate buffered saline (PBS) by serial dilutions. The diluted solutions were then seeded on a blood agar plate and incubated at 37°C for 24 hours. The results represent the number of Colony Forming Unit (CFU) counts on the blood agar plates.

The results are presented in Figure 1a. Figure 1a demonstrates that the bacteria killing concentration of oxacillin is much lower with grZ14s-nvCyc than with oxacillin alone, thereby demonstrating that grZ14s-nvCyc enhances the activity of the antibiotic.

As a comparison, a strain of methicillin sensitive *S. aureus* ATCC 25923 (5×10⁵ CFU/mL) was treated with oxacillin, alone or with the grZ14s-nvCyc (10nM). The results are presented in Figure 1b. Figure 1b demonstrates that there was no significant difference between the activity of oxacillin with or without grZ 14s-nvCyc. This demonstrates that the activity of a peptide of the present invention enhances the activity of an antibiotic against an antibiotic resistant species.

A similar assay was performed with *S. aureus* ATCC 43300 at a concentration of 7×10⁶ CFU/mL. The bacterial concentration in this assay is higher (∼7×10⁶ CFU/mL). The results are presented in Figure 2. Figure 2 demonstrates that the bacteria killing concentration of oxacillin on *S. aureus* ATCC 43300 MRSA strain is much lower with grZ14s-nvCyc than with oxacillin alone. With grZ14s-nvCyc, the oxacillin concentration needed to kill the bacteria (6.25µg/mL) is even lower than the oxacillin concentration needed to kill the methicillin sensitive *S. aureus* ATCC 25923 (12.5µg/mL) at the same bacterial concentration (∼7×10⁶ CFU/mL). This result demonstrates that a peptide of the present invention decreases the resistance of MRSA strain ATCC 43300 and makes it more sensitive to oxacillin than the sensitive *S. aureus* ATCC 25923 strain.

### Example 2 - Activity of oxacillin and a peptide comprising FDYDWY against methicillin resistant and methicillin sensitive S. aureus

*S. aureus* ATCC 33591 (MRSA strain) (5×10⁷ CFU/mL) and methicillin sensitive strain (MSSA) of *S. aureus* ATCC 25923 (1×10⁸ CFU/mL) were incubated in 1.5 mL Eppendorf tubes with either oxacillin alone or oxacillin with the grZ14s-nvCyc (10nM) for 24 hours at 37 °C with shaking (250 rpm). The solutions in the tubes were diluted ten-fold with PBS by serial dilutions. The diluted solutions were then seeded on a blood plate agar and incubated at 37°C for 24 hours.

The results are presented in Figure 3. As seen in Figure 3, the bacteria killing concentration of oxacillin against MRSA and MSSA was reduced to about half in the presence of a peptide of the invention. Thus, Figure 3 demonstrates that the activity of a peptide of the present invention enhances the activity of an antibiotic against an antibiotic resistant species and antibiotic sensitive species by decreasing the antibiotic specific and non- specific resistance mechanisms.

*S. aureus* ATCC 33591 (5×10⁵ CFU/mL) (MRSA strain) was incubated in 1.5 mL Eppendorf tubes with oxacillin, alone or with the grZ14s-nvCyc peptide (10nM), for 24 hours at 37°C with shaking (250 rpm). The lower concentration of the bacteria in the culture allows the bacteria to remain in the planktonic state and exhibit only specific resistance mechanisms. The solutions in the tubes were diluted ten-fold with phosphate buffered saline (PBS) by serial dilutions. The diluted solutions were then seeded on a blood plate agar and incubated at 37°C for 24 hours.

The results are presented in Figure 4a. Figure 4a demonstrates that bacteria killing concentration of oxacillin is much lower with grZ14s-nvCyc than with oxacillin alone, thereby demonstrating that grZ14s-nvCyc enhances the activity of the antibiotic. The activity demonstrates that the peptides of the invention are active against bacteria exhibiting specific resistance mechanisms.

As a comparison, a strain of methicillin sensitive *S. aureus* ATCC 25923 (1×10⁶ CFU/mL) at a bacterial concentration similar to the *S*. *aureus* ATCC 33591 concentration (MRSA strain (5×10⁵ CFU/mL) in figure 4a, was treated with either oxacillin, alone or with the grZ14s-nvCyc (10nM) peptide. The results are presented in Figure 4b. Figure 4b demonstrates that there was no significant difference between the activity of oxacillin with or without grZ14s-nvCyc. This demonstrates that the activity of a peptide of the present invention enhances the activity of an antibiotic against an antibiotic resistant species by decreasing specific antibiotic resistance.

### Example 3 - Activity of vancomycin in combination with a peptide comprising FDYDWY against resistant species of Enterococcus faecium

*Enterococcus faecium* ATCC 700221 (4×10² CFU/mL) (vancomycin, teicoplanin and tetracyclin resistant strain) was incubated in 1.5 mL Eppendorf tubes with either vancomycin, alone or with the grZ14s-nvCyc peptide (10nM), for 24 hours at 37°C with shaking (250 rpm). The solutions in the tubes were diluted ten-fold with phosphate buffered saline (PBS) by serial dilutions. The diluted solutions were then seeded on a blood plate agar and incubated at 37°C for 24 hours.

The results are presented in Figure 5. Figure 5 demonstrates that the bacteria killing concentration of vancomycin is much lower (about 50%) with grZ14s-nvCyc than with vancomycin alone, thereby demonstrating that grZ14s-nvCyc enhances the activity of the antibiotic on a vancomycin resistant strain.

### Example 4 - Activity of teicoplanin in combination with a peptide comprising FDYDWY against resistant species of Enterococcus faecium

*E. faecium* ATCC 700221 (2×10² CFU/mL) (vancomycin, teicoplanin and tetracyclin resistant strain) was incubated with either teicoplanin, alone or with the grZ14s-nvCyc peptide (10nM), for 24 hours at 37°C with shaking (250 rpm). The solutions were treated as described above.

The results are presented in Figure 6. Figure 6 demonstrates that the bacteria killing concentration of teicoplanin is much lower (about 60% lower) with grZ14s-nvCyc than with teicoplanin alone, thereby demonstrating that grZ14s-nvCyc enhances the activity of the antibiotic on teicoplanin resistant strain..

### Example 5 - Activity of ampicillin in combination with a peptide comprising FDYDWY against resistant species of Klebsiella pneumoniae

*Klebsiella pneumoniae* ATCC 700603 (MDR resistant strain: Resistant to ampicillin, aztreonam, cefoxitin, cefpodoxime, ceftazidime, chloramphenicol, piperacillin, tetracycline) (3×10⁵ CFU/mL) was incubated with either ampicillin, alone or with the grZ14s-nvCyc peptide (10nM), for 24 hours at 37°C with shaking (250 rpm). The solutions in the tubes were diluted ten-fold with phosphate buffered saline (PBS) by serial dilutions. The diluted solutions were then seeded on a blood plate agar and incubated at 37°C for 24 hours.

The results are presented in Figure 7. Figure 7 demonstrates that even very high concentrations of ampicillin alone could not achieve bacterial killing at a bacterial concentration of (3×10⁵ CFU/mL). However, combination of ampicillin with with grZ14s-nvCyc achieved bacteria killing activity at significantly lower concentrations (∼100% lower). This experiment demonstrates that grZ14s-nvCyc enhances the activity of the antibiotic on ampicillin resistant strains.

A similar assay was performed with *K. pneumoniae* at lower bacterial concentrations of 2×10³ CFU/mL. The results are presented in Figure 8. As seen in Figure 8, at the lower bacterial concentration, the antibiotic ampicillin achieves bacteria killing with the antibiotic alone. In addition, Figure 8 also demonstrates that the bacteria killing concentration of ampicillin is lower by 100 µg/mL with grZ14s-nvCyc than with ampicillin alone, thereby demonstrating that grZ14s-nvCyc enhances the activity of the antibiotic against an antibiotic resistant species exhibiting specific resistance.

### Example 6 - Activity of cefoxitin in combination with a peptide comprising FDYDWY against resistant species of Klebsiella pneumoniae

*K. pneumoniae* ATCC 700603 (MDR resistant strain: Resistant to ampicillin, aztreonam, cefoxitin, cefpodoxime, ceftazidime, chloramphenicol, piperacillin, tetracycline) (2×10⁶ CFU/mL) was incubated with either cefoxitin, alone or with the grZ14s-nvCyc peptide (10nM), for 24 hours at 37 °C with shaking (250 rpm). The solutions were then treated as described in the examples above.

The results are presented in Figure 9. Figure 9 demonstrates that the bacteria killing concentration of cefoxitin is much lower (>80% lower) with grZ14s-nvCyc than in cefoxitin alone, thereby demonstrating that grZ14s-nvCyc enhances the activity of the antibiotic against an antibiotic resistant species.

### Example 7 - Activity of carbapenems in combination with a peptide comprising FDYDWY against resistant species of K. pneumoniae (low and high bacterial concentration)

*K. pneumoniae* ATCC BAA® 1705 (3×10² CFU/mL) (carbapenemase positive strain) (carbapenem - imipenem and ertapenem, amikacin, aztreonam, levofloxacin and ciprofloxacin - resistant strain) was incubated with imipenem, alone or with the grZ14s-nvCyc peptide (10nM), for 24 hours at 37 °C with shaking (250 rpm). The solutions were then treated as described in the examples above.

The results are presented in Figure 10. Figure 10 demonstrates that the bacteria killing concentration of imipenem is much lower (>75% lower) with grZ14s-nvCyc than in imipenem alone, thereby demonstrating that grZ14s-nvCyc enhances the activity of the antibiotic against an antibiotic resistant species. The low concentration of bacteria indicates that the bacteria were present in their planktonic state. Thus, it is postulated that the peptide of the present invention is active against the specific resistance mechanisms of the bacteria.

Similarly, *K. pneumoniae* ATCC BAA® 1705 (1×10⁶ CFU/mL) was incubated with imipenem, alone or with grZ14s-nvCyc. The results are presented in Figure 11. Figure 11 demonstrates that the bacteria killing concentration of imipenem is much lower (>75% lower) with grZ14s-nvCyc than with imipenem alone, thereby demonstrating that grZ14s-nvCyc enhances the activity of the antibiotic against an antibiotic resistant species. The higher concentration of bacteria in the assay indicates that the bacteria could be in the planktonic or biofilm stage. The results demonstrate that the peptides of the invention are active against an antibiotic resistant species even at higher bacterial loads.

### Example 8 - Activity of mupirocin in combination with a peptide comprising FDYDWY against MRSA

*S. aureus* ATCC BAA® 1708 (MRSA strain, mupA positive) (1×10⁵ CFU/mL) was incubated with mupirocin, alone or with grZ14s-nvCyc as described in the Examples above. The results are presented in Figure 12. Figure 12 demonstrates that the bacterial load is reduced by at least one order of magnitude in the presence of a combination of mupirocin and grZ 14s-nvCyc.

### Example 9 - Activity of protein synthesis inhibitors in combination with a peptide comprising FDYDWY

*E. faecium* ATCC 700221 (1×10³ CFU/mL) (vancomycin, teicoplanin and tetracyclin resistant strain) was incubated with tetracyclin, alone or with grZ14s-nvCyc (10 mN), as described in the Examples above. The results are presented in Figure 13a. Figure 13a demonstrates that the bacteria killing concentration of tetracyclin is much lower (60% lower) with grZ14s-nvCyc than with tetracyclin alone against an antibiotic resistant species. Similarly, *E. faecium* ATCC 700221 (2×10⁵ CFU/mL) (vancomycin, teicoplanin and tetracyclin resistant strain) was incubated with tetracyclin, alone or with grZ14s-nvCyc (10 mN), as described in the Examples above. The results are presented in Figure 13b. Figure 13b demonstrates that the bacteria killing concentration of tetracyclin is much lower (∼70% lower) with grZ14s-nvCyc than with tetracyclin alone against an antibiotic resistant species.

Similarly, *K. pneumoniae* ATCC BAA® 1705 (1×10⁵ CFU/mL) was incubated with ciprofloxacin, alone or with grZ14s-nvCyc, as described in the Examples above. The results are presented in Figure 14. Figure 14 demonstrates that the bacteria killing concentration of ciprofloxacin is much lower (80% lower) with grZ14s-nvCyc than with ciprofloxacin alone against an antibiotic resistant species.

Similarly, *K. pneumoniae* ATCC 700603 (MDR resistant strain: Resistant to ampicillin, aztreonam, cefoxitin, cefpodoxime, ceftazidime, chloramphenicol, piperacillin, tetracycline) (4×10⁶ CFU/mL) was incubated with chloramphenicol, alone or with grZ14s-nvCyc, as described in the Examples above. The results are presented in Figure 15. Figure 15 demonstrates that chloramphenicol with grZ14s-nvCyc achieves bacteria killing at a concentration where chloramphenicol alone does not achieve bacteria killing.

Similarly, *K. pneumoniae* ATCC 700603 (MDR resistant strain: Resistant to ampicillin, aztreonam, cefoxitin, cefpodoxime, ceftazidime, chloramphenicol, piperacillin, tetracycline) (1×10³ CFU/mL) was incubated with chloramphenicol, alone or with grZ14s-nvCyc, as described in the Examples above. The results are presented in Figure 16. In this example, the bacterial concentration is lower, thus the antibiotic alone can achieve bacteria killing activity. Figure 16 demonstrates that bacteria killing concentration of chloramphenicol with grZ14s-nvCyc is much lower (20%) than with chloramphenicol alone against an antibiotic resistant species.

Similarly, *K. pneumoniae* ATCC BAA® 1705 (1×10⁶ CFU/mL) (carbapenem - imipenem and ertapenem, amikacin, aztreonam, levofloxacin and ciprofloxacin - resistant strain) was incubated with amikacin, alone or with grZ14s-nvCyc, as described in the Examples above. The results are presented in Figure 17. Figure 17 demonstrates that bacteria killing concentration of chloramphenicol with grZ14s-nvCyc is much lower (75%) than with chloramphenicol alone against an antibiotic resistant species.

### Example 10 - Activity of levofloxacin in combination with a peptide comprising FDYDWY

*K. pneumoniae* ATCC BAA® 1705 (2×10⁶ CFU/mL) (carbapenem - imipenem and ertapenem, amikacin, aztreonam, levofloxacin and ciprofloxacin - resistant strain) was incubated with levofloxacin, alone or with grZ14s-nvCyc, as described in the Examples above. The results are presented in Figure 18. Figure 18 demonstrates that bacteria killing concentration of levofloxacin with grZ14s-nvCyc is much lower (30%) than with levofloxacin alone against an antibiotic resistant species.

### Example 11 - Activity of aztreonam in combination with a peptide comprising FDYDWY

*K. pneumoniae* ATCC BAA® 1705 (1×10⁵ CFU/mL) (carbapenem - imipenem and ertapenem, amikacin, aztreonam, levofloxacin and ciprofloxacin - resistant strain) was incubated with aztreonam, alone or with grZ14s-nvCyc, as described in the Examples above. The results are presented in Figure 19. Figure 19 demonstrates that aztreonam with grZ14s-nvCyc achieves bacteria killing at a concentration (1000 µg/ml) where aztreonam alone does not achieve bacteria killing.

Similarly, *K. pneumoniae* ATCC BAA® 1705 (1×10³ CFU/mL) was incubated with aztreonam, alone or with grZ14s-nvCyc, as described in the Examples above. In this example, the bacterial concentration is lower, thus the antibiotic alone is able achieve bacteria killing activity. The results are presented in Figure 19b. Figure 19b demonstrates that bacteria killing concentration of aztreonam with grZ14s-nvCyc is much lower (80%) than with chloramphenicol alone against an antibiotic resistant species.

## Claims

1. An antibiotic and a cyclized peptide comprising a sequence SVHSFDYDWYNV for use in a method of increasing the effectiveness of an antibiotic against a bacterium, wherein the bacterium is an antibiotic-resistant species, comprising contacting the bacterium with the antibiotic and a cyclized peptide comprising a sequence SVHSFDYDWYNV, wherein the antibiotic and the peptide are administered to a subject in need thereof, wherein the subject is a mammal having a bacterial infection caused by the antibiotic resistant species.

2. The antibiotic and cyclized peptide comprising a sequence SVHSFDYDWYNV for use of claim 1, wherein the minimum bactericidal concentration of the antibiotic against the bacterium is decreased by at least 10%.

3. The antibiotic and cyclized peptide comprising a sequence SVHSFDYDWYNV for use of claim 1, wherein the peptide comprises up to 50 amino acids.

4. The antibiotic and cyclized peptide comprising a sequence SVHSFDYDWYNV for use of claim 1, wherein the antibiotic is selected from the group consisting of cell envelope antibiotics, nucleic acid inhibitors, and protein synthesis inhibitors.

5. The antibiotic and cyclized peptide comprising a sequence SVHSFDYDWYNV for use of claim 4, wherein the antibiotic is a cell envelope antibiotic selected from the group consisting of penicillins, cephalosporins, glycopeptides, and carbapenems.

6. The antibiotic and cyclized peptide comprising a sequence SVHSFDYDWYNV for use of claim 5, wherein the antibiotic is a penicillin selected from the group consisting of oxacillin, methicillin, amoxicillin, ampicillin, cloxacillin, dicloxacillin, carbenicillin, ticarcillin, and piperacillin;
the antibiotic and cyclized peptide comprising a sequence SVHSFDYDWYNV for use of claim 5, wherein the antibiotic is a cephalosporin selected from the group consisting of cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, carbacephems: loracarbef, cefbuperazone, cefmetazole, cefminox, cefotetan, cefoxitin, cefotiam, cefclidine, cefepime, cefluprenam, cefoselis, cefozopran, cefpirome, cefquinome, flomoxef, ceftobiprole, and ceftaroline;
the antibiotic and cyclized peptide comprising a sequence SVHSFDYDWYNV for use of claim 5, wherein the antibiotic is a glycopeptide selected from the group consisting of vancomycin and teicoplanin; or
the antibiotic and cyclized peptide comprising a sequence SVHSFDYDWYNV for use of claim 5, wherein the antibiotic is a carbapenem selected from the group consisting of imipenem, morepenem, ertapenem, doripenem, panipenem, and biapenem.

7. The antibiotic and cyclized peptide comprising a sequence SVHSFDYDWYNV for use of claim 4, wherein the antibiotic is a nucleic acid inhibitor selected from the group consisting of fluoroquinolones.

8. The antibiotic and cyclized peptide comprising a sequence SVHSFDYDWYNV for use of claim 7, wherein the floroquinolone is ciprofloxacin.

9. The antibiotic and peptide comprising a sequence SVHSFDYDWYNV for use of claim 4, wherein the antibiotic is a protein synthesis inhibitor selected from the group consisting of chloramphenicol and amikacin.

10. The antibiotic and peptide comprising a sequence SVHSFDYDWYNV for use of any one of the preceding claims, wherein the peptide has the sequence CSVHSFDYDWYNVC.

11. The antibiotic and peptide comprising a sequence SVHSFDYDWYNV for use of any one of claims 1 to 10, wherein the peptide is modified with a cysteine at the C-terminal and a cysteine at the N-terminal, wherein the C- and N-terminals are S-S bridged.

12. The antibiotic and peptide comprising a sequence SVHSFDYDWYNV for use of claim 1, wherein the mammal is a human.

13. The antibiotic and peptide comprising a sequence SVHSFDYDWYNV for use of claim 1, wherein the bacterium is a gram positive species or a gram negative species.

14. The antibiotic and peptide comprising a sequence SVHSFDYDWYNV for use of claim 13, wherein the Gram-positive bacterium is Actinomyces spp., Bacillus anthracis, Bifidobacterium spp., Clostridium botulinum, Clostridium perfringens, Clostridium spp., Clostridium tetani, Corynebacterium diphtheriae, Corynebacterium jeikeium, Enterococcus faecalis, Enterococcus faecium, Erysipelothrix rhusiopathiae, Eubacterium spp., Gardnerella vaginalis, Gemella morbillorum, Leuconostoc spp., Mycobacterium abscessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia spp., Peptococcus niger, Peptostreptococcus spp., Proprionibacterium spp., Sarcina lutea, Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae (group B streptococcus), Streptococcus anginosus, Streptococcus bovis, Streptococcus canis, Streptococcus equi, Streptococcus milleri, Streptococcus mitior, Streptococcus mutans, Streptococcus pneumoniae, Streptococcus pyogenes (group A streptococcus), Streptococcus salivarius or Streptococcus sanguis.

15. The antibiotic and peptide comprising a sequence SVHSFDYDWYNV for use of claim 13, wherein the Gram-negative bacterium is Acinetobacter calcoaceticus, Acinetobacter baumannii, Actinobacillus actinomycetemcomitans, Aeromonas hydrophila, Alcaligenes xylosoxidans, Bacteroides, Bacteroides fragilis, Bartonella bacilliformis, Bordetella spp., Borrelia burgdorferi, Branhamella catarrhalis, Brucella spp., Campylobacter spp., Chalmydia pneumoniae, Chlamydia psittaci, Chlamydia trachomatis, Chromobacterium violaceum, Citrobacter spp., Eikenella corrodens, Enterobacter aerogenes, Escherichia coli, Flavobacterium meningosepticum, Fusobacterium spp., Haemophilus influenzae, Haemophilus spp., Helicobacter pylori, Klebsiella pneumoniae, Klebsiella spp., Legionella spp., Leptospira spp., Moraxella catarrhalis, Morganella morganii, Mycoplasma pneumoniae, Neisseria gonorrhoeae, Neisseria meningitidis, Pasteurella multocida, Plesiomonas shigelloides, Prevotellaspp., Proteus spp., Providencia rettgeri, Pseudomonas aeruginosa, Pseudomonas spp., Rickettsia prowazekii, Rickettsia rickettsii, Rochalimaea spp., Salmonella spp., Salmonella typhi, Serratia marcescens, Shigella spp., Shigella sonnei, Treponema carateum, Treponema pallidum, Treponema pallidum endemicum, Treponema pertenue, Veillonella spp.,Vibrio cholerae, Vibrio vulnificus, Yersinia enterocolitica or Yersinia pestis.

16. The antibiotic and peptide comprising a sequence SVHSFDYDWYNV for use of claim 1, wherein the bacterium is Staphylococcus aureus, Enterococcus faecium or Klebsiella pneumonia.

17. The antibiotic and peptide comprising a sequence SVHSFDYDWYNV for use of claim 16, wherein the Staphylococcus aureus is a methicillin resistant species (MRSA);
the antibiotic and peptide comprising a sequence SVHSFDYDWYNV for use of claim 16, wherein the Enterococcus faecium is a vancomycin-resistant species; or
wherein the Enterococcus faecium is a teicoplanin-resistant species; or
wherein the Enterococcus faecium is a vancomycin- and teicoplanin-resistant species; or
wherein the Enterococcus faecium is resistant to the tetracyclin group of antibiotics; or
the antibiotic and peptide comprising a sequence SVHSFDYDWYNV for use of claim 16, wherein the Klebsiella pneumonia is resistant to the penicillin group of antibiotics; or
wherein the Klebsiella pneumoniae is resistant to the cephalosporin group of antibiotics; or
wherein the Klebsiella pneumonia is resistant to the carbapenem group of antibiotics; or
wherein the Klebsiella pneumonia is resistant to the fluoroquinolone group of antibiotics; or wherein the Klebsiella pneumonia is resistant to protein synthesis inhibitor antibiotics; or
wherein the Klebsiella pneumonia is resistant to ampicillin, aztreonam, cefoxitin, cefpodoxime, ceftazidime, chloramphenicol, piperacillin, and tetracycline; or wherein the Klebsiella pneumonia is resistant to carbapenem - imipenem and ertapenem, amikacin, aztreonam, levofloxacin, and ciprofloxacin.

## Patentansprüche

1. Antibiotikum und cyclisiertes Peptid, das eine Sequenz SVHSFDYDWYNV umfasst, zur Verwendung in einem Verfahren zur Erhöhung der Wirksamkeit eines Antibiotikums gegen ein Bakterium, wobei das Bakterium eine Antibiotika-resistente Art ist, umfassend das Inkontaktbringen des Bakteriums mit dem Antibiotikum und einem cyclisierten Peptid, das eine Sequenz SVHSFDYDWYNV umfasst, wobei das Antibiotikum und das Peptid einem Individuum, das dessen bedarf, verabreicht werden, wobei das Individuum ein Säuger ist, der eine bakterielle Infektion hat, die von der Antibiotika-resistenten Art verursacht wurde.

2. Antibiotikum und cyclisiertes Peptid, das eine Sequenz SVHSFDYDWYNV umfasst, zur Verwendung nach Anspruch 1, wobei die minimale bakterizide Konzentration des Antibiotikums gegen das Bakterium um mindestens 10% verringert ist.

3. Antibiotikum und cyclisiertes Peptid, das eine Sequenz SVHSFDYDWYNV umfasst, zur Verwendung nach Anspruch 1, wobei das Peptid bis zu 50 Aminosäuren umfasst.

4. Antibiotikum und cyclisiertes Peptid, das eine Sequenz SVHSFDYDWYNV umfasst, zur Verwendung nach Anspruch 1, wobei das Antibiotikum ausgewählt ist aus der Gruppe bestehend aus Zellwand-Antibiotika (cell envelope antibiotics), Nucleinsäureinhibitoren und Proteinsyntheseinhibitoren.

5. Antibiotikum und cyclisiertes Peptid, das eine Sequenz SVHSFDYDWYNV umfasst, zur Verwendung nach Anspruch 4, wobei das Antibiotikum ein Zellwand-Antibiotikum ausgewählt aus der Gruppe bestehend aus Penicillinen, Cephalosporinen, Glycopeptiden und Carbapenemen ist.

6. Antibiotikum und cyclisiertes Peptid, das eine Sequenz SVHSFDYDWYNV umfasst, zur Verwendung nach Anspruch 5, wobei das Antibiotikum ein Penicillin ausgewählt aus der Gruppe bestehend aus Oxacillin, Methicillin, Amoxicillin, Ampicillin, Cloxacillin, Dicloxacillin, Carbenicillin, Ticarcillin und Piperacilllin ist;
Antibiotikum und cyclisiertes Peptid, das eine Sequenz SVHSFDYDWYNV umfasst, zur Verwendung nach Anspruch 5, wobei das Antibiotikum ein Cephalosporin ausgewählt aus der Gruppe bestehend aus Cefacetril, Cefadroxil, Cephalexin, Cefaloglycin, Cefalonium, Cefaloridin, Cefalotin, Cefapirin, Cefatrizin, Cefazaflur, Cefazedon, Cefazolin, Cefradrin, Cefroxadin, Ceftezol, Cefaclor, Cefonicid, Cefprozil, Cefuroxim, Cefuzonam, Cefmetazol, Cefotetan, den Carbacephemen: Loracarbef, Cefbuperazon, Cefmetazol, Cefminox, Cefotetan, Cefoxitin, Cefotiam, Cefclidin, Cefepim, Cefluprenam, Cefoselis, Cefozopran, Cefpirom, Cefquinom, Flomoxef, Ceftobiprol und Ceftarolin ist;
Antibiotikum und cyclisiertes Peptid, das eine Sequenz SVHSFDYDWYNV umfasst, zur Verwendung nach Anspruch 5, wobei das Antibiotikum ein Glycopeptid ausgewählt aus der Gruppe bestehend aus Vancomycin und Teicoplanin ist; oder
Antibiotikum und cyclisiertes Peptid, das eine Sequenz SVHSFDYDWYNV umfasst, zur Verwendung nach Anspruch 5, wobei das Antibiotikum ein Carbapenem ausgewählt aus der Gruppe bestehend aus Imipenem, Morepenem, Ertapenem, Doripenem, Panipenem und Biapenem ist.

7. Antibiotikum und cyclisiertes Peptid, das eine Sequenz SVHSFDYDWYNV umfasst, zur Verwendung nach Anspruch 4, wobei das Antibiotikum ein Nucleinsäureinhibitor ausgewählt aus der Gruppe bestehend aus Fluorchinolonen ist.

8. Antibiotikum und cyclisiertes Peptid, das eine Sequenz SVHSFDYDWYNV umfasst, zur Verwendung nach Anspruch 7, wobei das Fluorchinolon Ciprofloxacin ist.

9. Antibiotikum und Peptid, das eine Sequenz SVHSFDYDWYNV umfasst, zur Verwendung nach Anspruch 4, wobei das Antibiotikum ein Proteinsyntheseinhibitor ausgewählt aus der Gruppe bestehend aus Chloramphenicol und Amikacin ist.

10. Antibiotikum und Peptid, das eine Sequenz SVHSFDYDWYNV umfasst, zur Verwendung nach einem der vorangehenden Ansprüche, wobei das Peptid die Sequenz CSVHSFDYDWYNVC aufweist.

11. Antibiotikum und Peptid, das eine Sequenz SVHSFDYDWYNV umfasst, zur Verwendung nach einem der Ansprüche 1 bis 10, wobei das Peptid mit einem Cystein am C-Terminus und einem Cystein am N-Terminus modifiziert ist, wobei der C-Terminus und der N-Terminus über eine S-S-Brücke verknüpft sind.

12. Antibiotikum und Peptid, das eine Sequenz SVHSFDYDWYNV umfasst, zur Verwendung nach Anspruch 1, wobei Säuger ein Mensch ist.

13. Antibiotikum und Peptid, das eine Sequenz SVHSFDYDWYNV umfasst, zur Verwendung nach Anspruch 1, wobei das Bakterium eine grampositive Art oder eine gramnegative Art ist.

14. Antibiotikum und Peptid, das eine Sequenz SVHSFDYDWYNV umfasst, zur Verwendung nach Anspruch 13, wobei das grampositive Bakterium *Actinomyces spp., Bacillus anthracis, Bifidobacterium spp., Clostridium botulinum, Clostridium perfringens, Clostridium spp., Clostridium tetani, Corynebacterium diphtheriae, Corynebacterium jeikeium, Enterococcus faecalis, Enterococcus faecium, Erysipelothrix rhusiopathiae, Eubacterium spp., Gardnerella vaginalis, Gemella morbillorum, Leuconostoc spp., Mycobacterium abscessus, Mycobacterium avium-Komplex, Mycobacterium chelonae*, *Mycobacterium fortuitum, Mycobacterium haemophilium, Mycobacterium kansasii, Mycobacterium leprae, Mycobacterium marinum, Mycobacterium scrofulaceum*, *Mycobacterium smegmatis, Mycobacterium terrae, Mycobacterium tuberculosis, Mycobacterium ulcerans, Nocardia spp., Peptococcus niger, Peptostreptococcus spp*., *Proprionibacterium spp., Sarcina lutea, Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (Gruppe B-Streptococcus), *Streptococcus anginosus, Streptococcus bovis, Streptococcus canis, Streptococcus equi, Streptococcus milleri, Streptococcus mitior, Streptococcus mutans*, *Streptococcus pneumoniae, Streptococcus pyogenes* (Gruppe A-Streptococcus), *Streptococcus salivarius* oder *Streptococcus sanguis* ist.

15. Antibiotikum und Peptid, das eine Sequenz SVHSFDYDWYNV umfasst, zur Verwendung nach Anspruch 13, wobei das gramnegative Bakterium *Acinetobacter calcoaceticus, Acinetobacter baumannii, Actinobacillus actinomycetemcomitans, Aeromonas hydrophila, Alcaligenes xylosoxidans, Bacteroides, Bacteroides fragilis*, *Bartonella bacilliformis, Bordetella spp., Borrelia burgdorferi, Branhamella catarrhalis, Brucella spp., Campylobacter spp., Chalmydia pneumoniae, Chlamydia psittaci, Chlamydia trachomatis, Chromobacterium violaceum, Citrobacter spp., Eikenella corrodens, Enterobacter aerogenes, Escherichia coli, Flavobacterium meningosepticum, Fusobacterium spp., Haemophilus influenzae, Haemophilus spp., Helicobacter pylori, Klebsiella pneumoniae, Klebsiella spp., Legionella spp., Leptospira spp., Moraxella catarrhalis, Morganella morganii, Mycoplasma pneumoniae, Neisseria gonorrhoeae, Neisseria meningitidis, Pasteurella multocida, Plesiomonas shigelloides, Prevotellaspp., Proteus spp., Providencia rettgeri, Pseudomonas aeruginosa, Pseudomonas spp., Rickettsia prowazekii, Rickettsia rickettsii, Rochalimaea spp., Salmonella spp., Salmonella typhi, Serratia marcescens, Shigella spp., Shigella sonnei, Treponema carateum, Treponema pallidum, Treponema pallidum endemicum, Treponema pertenue, Veillonella spp., Vibrio cholerae, Vibrio vulnificus, Yersinia enterocolitica* oder *Yersinia pestis* ist.

16. Antibiotikum und Peptid, das eine Sequenz SVHSFDYDWYNV umfasst, zur Verwendung nach Anspruch 1, wobei das Bakterium *Staphylococcus aureus, Enterococcus faecium* oder *Klebsiella pneumonia* ist.

17. Antibiotikum und Peptid, das eine Sequenz SVHSFDYDWYNV umfasst, zur Verwendung nach Anspruch 16, wobei das *Staphylococcus* aureus-Bakterium eine Methicillin-resistente Art (methicillin-resistant *Staphilococcus aureus*; MRSA) ist;
Antibiotikum und Peptid, das eine Sequenz SVHSFDYDWYNV umfasst, zur Verwendung nach Anspruch 16, wobei das *Enterococcus* faecium-Bakterium eine Vancomycin-resistente Art ist; oder
wobei das *Enterococcus* faecium-Bakterium eine Teicoplanin-resistente Art ist; oder
wobei das *Enterococcus* faecium-Bakterium eine Vancomycin- und Teicoplanin-resistente Art ist; oder
wobei das *Enterococcus faecium*-Bakterium gegenüber der Tetracyclin-Gruppe von Antibiotika resistent ist; oder
Antibiotikum und Peptid, das eine Sequenz SVHSFDYDWYNV umfasst, zur Verwendung nach Anspruch 16, wobei das *Klebsiella pneumonia-Bakterium* gegenüber der Penicillin-Gruppe von Antibiotika resistent ist; oder
wobei das *Klebsiella* pneumoniae-Bakterium gegenüber der Cephalosporin-Gruppe von Antibiotika resistent ist; oder
wobei das *Klebsiella pneumonia-Bakterium* gegenüber der Carbapenem-Gruppe von Antibiotika resistent ist;
wobei das *Klebsiella pneumonia*-Bakterium gegenüber der Fluorochinolon-Gruppe von Antibiotika resistent ist; oder wobei das *Klebsiella pneumonia-*Bakterium gegenüber der Proteinsynthese-Inhibitor-Antibiotika resistent ist; oder
wobei das *Klebsiella pneumonia*-Bakterium gegenüber Ampicillin, Aztreonam, Cefoxitin, Cefpodoxim, Ceftazidim, Chloramphenicol Piperacillin und Tetracyclin resistent ist; oder
wobei das *Klebsiella pneumonia-Bakterium* gegenüber Carbapenem - Imipenem und Ertapenem, Amikacin, Aztreonam, Levofloxacin und Ciprofloxacin resistent ist.

## Revendications

1. Antibiotique et peptide cyclisé comprenant la séquence SVHSFDYDWYNV pour une utilisation dans une méthode d'augmentation de l'efficacité d'un antibiotique contre une bactérie, dans lesquels la bactérie est une espèce résistante aux antibiotiques, comprenant la mise en contact de la bactérie avec un antibiotique et un peptide cyclisé comprenant la séquence SVHSFDYDWYNV, dans lesquels l'antibiotique et le peptide sont administrés à un sujet en ayant besoin, dans lesquels le sujet est un mammifère ayant une infection bactérienne provoquée par l'espèce résistante aux antibiotiques.

2. Antibiotique et peptide cyclisé comprenant la séquence SVHSFDYDWYNV pour une utilisation selon la revendication 1, dans lesquels la concentration bactéricide minimale de l'antibiotique contre la bactérie est réduite d'au moins 10 %.

3. Antibiotique et peptide cyclisé comprenant la séquence SVHSFDYDWYNV pour une utilisation selon la revendication 1, dans lesquels le peptide comprend jusqu'à 50 acides aminés.

4. Antibiotique et peptide cyclisé comprenant la séquence SVHSFDYDWYNV pour une utilisation selon la revendication 1, dans lesquels l'antibiotique est choisi dans le groupe constitué par les antibiotiques d'enveloppe cellulaire, les antibiotiques d'acide nucléique, et les inhibiteurs de synthèse de protéine.

5. Antibiotique et peptide cyclisé comprenant la séquence SVHSFDYDWYNV pour une utilisation selon la revendication 4, dans lesquels l'antibiotique est un antibiotique d'enveloppe cellulaire choisi dans le groupe constitué par les pénicillines, les céphalosporines, les glycopeptides, et les carbapénèmes.

6. Antibiotique et peptide cyclisé comprenant la séquence SVHSFDYDWYNV pour une utilisation selon la revendication 5, dans lesquels l'antibiotique est une pénicilline choisie dans le groupe constitué par l'oxacilline, la méthicilline, l'amoxicilline, l'ampicilline, la cloxacilline, la dicloxacilline, la carbénicilline, la ticarcilline, et la pipéracilline ;
les antibiotique et peptide cyclisé comprenant la séquence SVHSFDYDWYNV pour une utilisation selon la revendication 5, dans lesquels l'antibiotique est une céphalosporine choisie dans le groupe constitué par le céfacétrile, le céfadroxil, la céphalexine, la céfaloglycine, le céfalonium, la céfaloridine, la céfalotine, la céfapirine, la céfatrizine, le céfazaflur, la céfazédone, la céfazoline, la céfradine, la céfroxadine, le ceftézole, le céfaclor, le céfonicid, le cefprozil, la céfuroxime, le céfuzonam, le cefmétazole, le céfotétan, les carbacéphèmes ; le loracarbef, la cefbupérazone, le cefmétazole, le cefminox, le céfotétan, la céfoxitine, le céfotiam, la cefclidine, la céfépime, le céfluprénam, le céfosélis, le céfozopran, la cefpirome, la cefquinome, le flomoxef, le ceftobiprole, et la ceftaroline ;
les antibiotique et peptide cyclisé comprenant la séquence SVHSFDYDWYNV pour une utilisation selon la revendication 5, dans lesquels l'antibiotique est un glycopeptide choisi dans le groupe constitué par la vancomycine et la téicoplanine ; ou
les antibiotique et peptide cyclisé comprenant la séquence SVHSFDYDWYNV pour une utilisation selon la revendication 5, dans lesquels l'antibiotique est un carbapénème choisi dans le groupe constitué par l'imipénème, le morépénème, l'ertapénème, le doripénème, le panipénème, et le biapénème.

7. Antibiotique et peptide cyclisé comprenant la séquence SVHSFDYDWYNV pour une utilisation selon la revendication 4, dans lesquels l'antibiotique est un inhibiteur d'acide nucléique choisi dans le groupe constitué par les fluoroquinolones.

8. Antibiotique et peptide cyclisé comprenant la séquence SVHSFDYDWYNV pour une utilisation selon la revendication 7, dans lesquels la fluoroquinolone est la ciprofloxacine.

9. Antibiotique et peptide comprenant une séquence SVHSFDYDWYNV pour une utilisation selon la revendication 4, dans lesquels l'antibiotique est un inhibiteur de la synthèse de protéine choisi dans le groupe constitué par le chloramphénicol et l'amikacine.

10. Antibiotique et peptide comprenant une séquence SVHSFDYDWYNV pour une utilisation selon l'une quelconque des revendications précédentes, dans lesquels le peptide a la séquence CSVHSFDYDWYNVC.

11. Antibiotique et peptide comprenant une séquence SVHSFDYDWYNV pour une utilisation selon l'une quelconque des revendications 1 à 10, dans lesquels le peptide est modifié par une cystéine à l'extrémité C et une cystéine à l'extrémité N, dans lesquels les extrémités C et N sont pontées par un pont S-S.

12. Antibiotique et peptide comprenant une séquence SVHSFDYDWYNV pour une utilisation selon la revendication 1, dans lesquels le mammifère est un humain.

13. Antibiotique et peptide comprenant une séquence SVHSFDYDWYNV pour une utilisation selon la revendication 1, dans lesquels la bactérie est une espèce Gram positive ou une espèce Gram négative.

14. Antibiotique et peptide comprenant une séquence SVHSFDYDWYNV pour une utilisation selon la revendication 13, dans lesquels la bactérie Gram positive est Actinomyces spp., Bacillus anthracis, Bifidobacterium spp., Clostridium botulinum, Clostridium perfringens, Clostridium spp., Clostridium tetani, Corynebacterium diphtheriae, Corynebacterium jeikeium, Enterococcus faecalis, Enterococcus faecium, Erysipelothrix rhusiopathiae, Eubacterium spp., Gardnerella vaginalis, Gemella morbillorum, Leuconostoc spp., Mycobacterium abscessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia spp., Peptococcus niger, Peptostreptococcus spp., Proprionibacterium spp., Sarcina lutea, Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae (Streptococcus du groupe B), Streptococcus anginosus, Streptococcus bovis, Streptococcus canis, Streptococcus equi, Streptococcus milleri, Streptococcus mitior, Streptococcus mutans, Streptococcus pneumoniae, Streptococcus pyogenes (Streptococcus du groupe A), Streptococcus salivarius ou Streptococcus sanguis.

15. Antibiotique et peptide comprenant une séquence SVHSFDYDWYNV pour une utilisation selon la revendication 13, dans lesquels la bactérie Gram négative est Acinetobacter calcoaceticus, Acinetobacter baumannii, Actinobacillus actinomycetemcomitans, Aeromonas hydrophila, Alcaligenes xylosoxidans, Bacteroides, Bacteroides fragilis, Bartonella bacilliformis, Bordetella spp., Borrelia burgdorferi, Branhamella catarrhalis, Brucella spp., Campylobacter spp., Chalmydia pneumoniae, Chlamydia psittaci, Chlamydia trachomatis, Chromobacterium violaceum, Citrobacter spp., Eikenella corrodens, Enterobacter aerogenes, Escherichia coli, Flavobacterium meningosepticum, Fusobacterium spp., Haemophilus influenzae, Haemophilus spp., Helicobacter pylori, Klebsiella pneumoniae, Klebsiella spp., Legionella spp., Leptospira spp., Moraxella catarrhalis, Morganella morganii, Mycoplasma pneumoniae, Neisseria gonorrhoeae, Neisseria meningitidis, Pasteurella multocida, Plesiomonas shigelloides, Prevotellaspp., Proteus spp., Providencia rettgeri, Pseudomonas aeruginosa, Pseudomonas spp., Rickettsia prowazekii, Rickettsia rickettsii, Rochalimaea spp., Salmonella spp., Salmonella typhi, Serratia marcescens, Shigella spp., Shigella sonnei, Treponema carateum, Treponema pallidum, Treponema pallidum endemicum, Treponema pertenue, Veillonella spp., Vibrio cholerae, Vibrio vulnificus, Yersinia enterocolitica ou Yersinia pestis.

16. Antibiotique et peptide comprenant une séquence SVHSFDYDWYNV pour une utilisation selon la revendication 1, dans lesquels la bactérie est Staphylococcus aureus, Enterococcus faecium ou Klebsiella pneumonia.

17. Antibiotique et peptide comprenant une séquence SVHSFDYDWYNV pour une utilisation selon la revendication 16, dans lesquels le Staphylococcus aureus est une espèce résistante à la méthicilline (SARM) ;
les antibiotique et peptide comprenant une séquence SVHSFDYDWYNV pour une utilisation selon la revendication 16, dans lesquels l'Enterococcus faecium est une espèce résistante à la vancomycine ; ou
dans lesquels l'Enterococcus faecium est une espèce résistante à la téicoplanine ; ou
dans lesquels l'Enterococcus faecium est une espèce résistante à la vancomycine et à la téicoplanine ; ou
dans lesquels l'Enterococcus faecium est résistant au groupe d'antibiotiques tétracyclines ; ou
les antibiotique et peptide comprenant une séquence SVHSFDYDWYNV pour une utilisation selon la revendication 16, dans lesquels la Klebsiella pneumonia est résistante au groupe d'antibiotiques pénicillines ; ou
dans lesquels la Klebsiella pneumoniae est résistante au groupe d'antibiotiques céphalosporines ; ou
dans lesquels la Klebsiella pneumonia est résistante au groupe d'antibiotiques carbapénèmes ; ou
dans lesquels la Klebsiella pneumonia est résistante au groupe d'antibiotiques fluoroquinolones ; ou dans lesquels la Klebsiella pneumonia est résistante aux antibiotiques inhibiteurs de la synthèse de protéine ; ou
dans lesquels la Klebsiella pneumonia est résistante à l'ampicilline, à l'aztréonam, à la céfoxitine, à la cefpodoxime, à la ceftazidime, au chloramphénicol, à la pipéracilline, et à la tétracycline ; ou
dans lesquels la Klebsiella pneumonia est résistante au carbapénème-imipénème et à l'ertapénème, à l'amikacine, à l'aztréonam, à la lévofloxacine, et à la ciprofloxacine.
